# EUROPEAN PATENT SPECIFICATION

(11) **EP 4 218 537 B1**
(45) Date of publication and mention of the grant of the patent: **29.04.2026**
(21) Application number: 23153512.1
(22) Date of filing: 26.01.2023
(51) Int. Cl.: A61B 1/06, G02B 23/24

(54) **ENDOSCOPE LIGHT SOURCE DEVICE, ENDOSCOPE SYSTEM, AND METHOD OF CHANGING ILLUMINATION LIGHT IN ENDOSCOPE LIGHT SOURCE DEVICE**
ENDOSKOPLICHTQUELLENVORRICHTUNG, ENDOSKOPSYSTEM UND VERFAHREN ZUM ÄNDERN DES BELEUCHTUNGSLICHTS IN DER ENDOSKOPLICHTQUELLENVORRICHTUNG
DISPOSITIF DE SOURCE DE LUMIÈRE D'ENDOSCOPE, SYSTÈME D'ENDOSCOPE ET PROCÉDÉ DE CHANGEMENT DE LUMIÈRE D'ÉCLAIRAGE DANS UN DISPOSITIF DE SOURCE DE LUMIÈRE D'ENDOSCOPE

(30) Priority: 27.01.2022 JP 2022010760
(43) Date of publication of application: 02.08.2023
(73) Proprietor: FUJIFILM Corporation, Tokyo 106-8620 (JP)
(72) Inventor: TABE, Hiroaki, Kanagawa (JP); MORIMOTO, Yoshinori, Kanagawa (JP)
(74) Representative: Klunker IP Patentanwälte PartG mbB

(56) References cited:
- EP-A1- 2 806 205
- JP-A- H11 225 952
- US-A1- 2011 235 324
- US-A1- 2015 065 802

## Description

### BACKGROUND OF THE INVENTION

### 1. Field of the Invention

The present invention relates to an endoscope light source device, an endoscope system, and a method of changing illumination light in the endoscope light source device.

### 2. Description of the Related Art

In a medical field, a diagnosis using an endoscope light source device and an endoscope system including an endoscope is widely performed. In the diagnosis using the endoscopic system, an image (hereinafter, referred to as an endoscopic image) obtained by imaging an observation target with an endoscope through image enhanced endoscopy (IEE) in which illumination light emitted from an endoscope light source device is devised may be used to obtain various types of diagnostic support information regarding a surface structure of the observation target, a surface layer of the mucous membrane, or the like. In the diagnosis using image enhanced endoscopy, there are cases where an appropriate diagnosis can be made by acquiring a plurality of types of endoscopic images obtained by a plurality of types of illumination light and the like and comparing or superimposing these endoscopic images in detail.

There is an endoscope light source device capable of stably obtaining illumination light having a target emission spectrum by using a plurality of semiconductor light sources including a fluorescent semiconductor light source with respect to a plurality of types of illumination light with simple control (WO2015/016013A and JP2015-070946A).

US 2011/235324 Al discloses a light source device for endoscopic or exoscopic applications includes a flat first light source, a second light source, an illuminating beam path that is configured to provide a first light beam emanating from the first light source for an endoscopic or exoscopic application, and a coupling device to couple a second light beam from the second light source into the illuminating beam path, whereby the coupling device is configured in such a way that at the coupling site the cross-section surface of the second light beam is smaller than the cross-section surface of the first light beam.

US 2015/065802 discloses an endoscope light source system having an illuminator with multiple semiconductor light sources that emit different wavelengths, the illuminator being attachable to an integral optical coupling device that combines their light to generate white illumination light for endoscopic applications. An additional violet semiconductor light source forms part of the illuminator, an associated dichroic mirror forms part of the intergral optical coupling device to combine the white light and the violet light.

### SUMMARY OF THE INVENTION

In the endoscope light source device in WO2015/016013A and JP2015-070946A, an amount of light of each color emitted from each of a plurality of semiconductor light sources is controlled, and light of each color is multiplexed by using an optical member such as a dichroic filter such that a multicolored light source that generates illumination light of a plurality of colors is realized. However, as a method of generating illumination light of a plurality of colors, operating a semiconductor light source such as adding a semiconductor light source of another color to a plurality of semiconductor light sources, or changing one of a plurality of semiconductor light sources to a semiconductor light source of another color is hardly realized because the entire optical system needs to be redesigned.

An object of the present invention is to provide an endoscope light source device and an endoscope system which easily realize addition or change of a light source, and a method of changing illumination light in the endoscope light source device.

The invention is set out in the independent claims. The features of the dependent patent claims relate to advantageous embodiments and extensions of the invention.

According to the present invention, it is possible to easily realize addition or change of a light source.

### BRIEF DESCRIPTION OF THE DRAWINGS

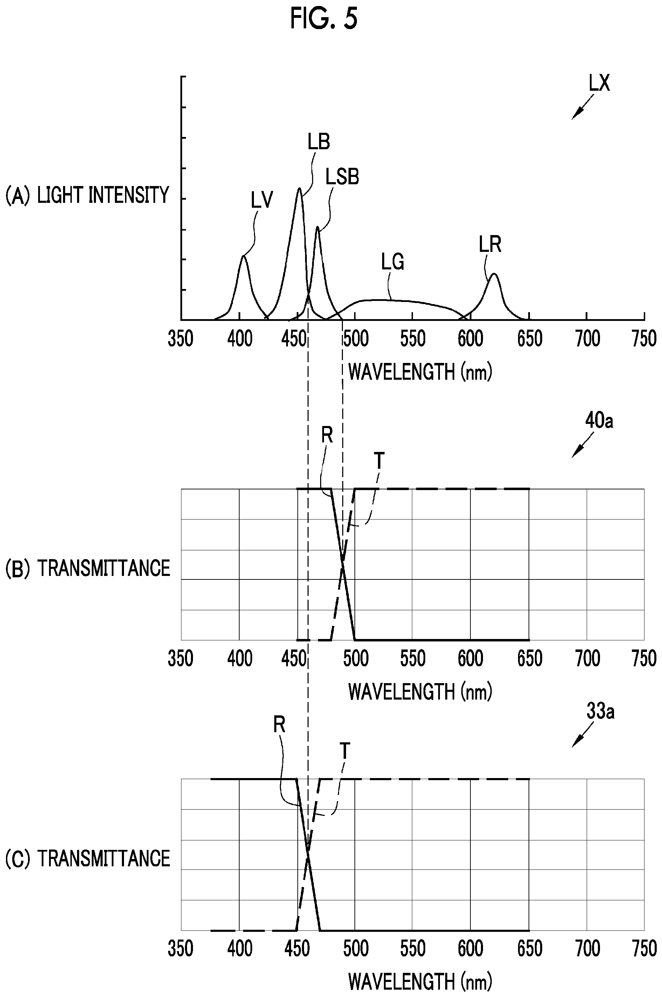

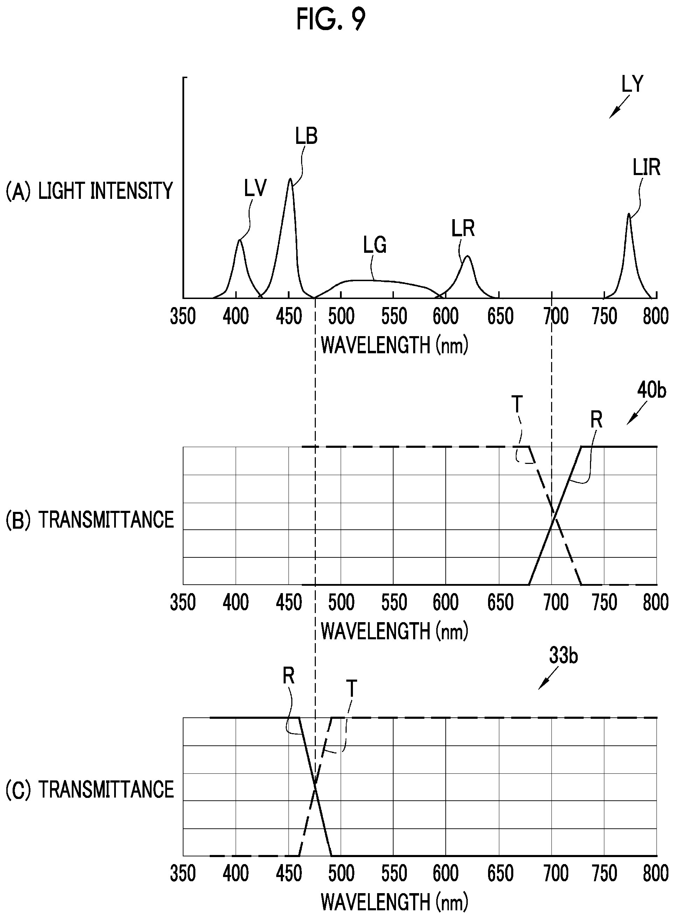

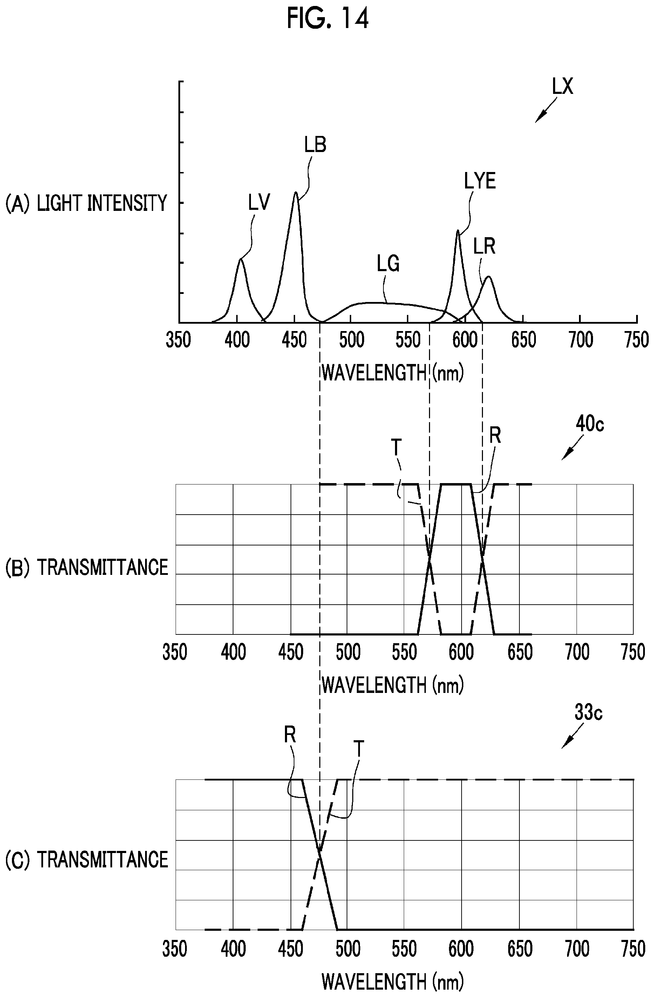

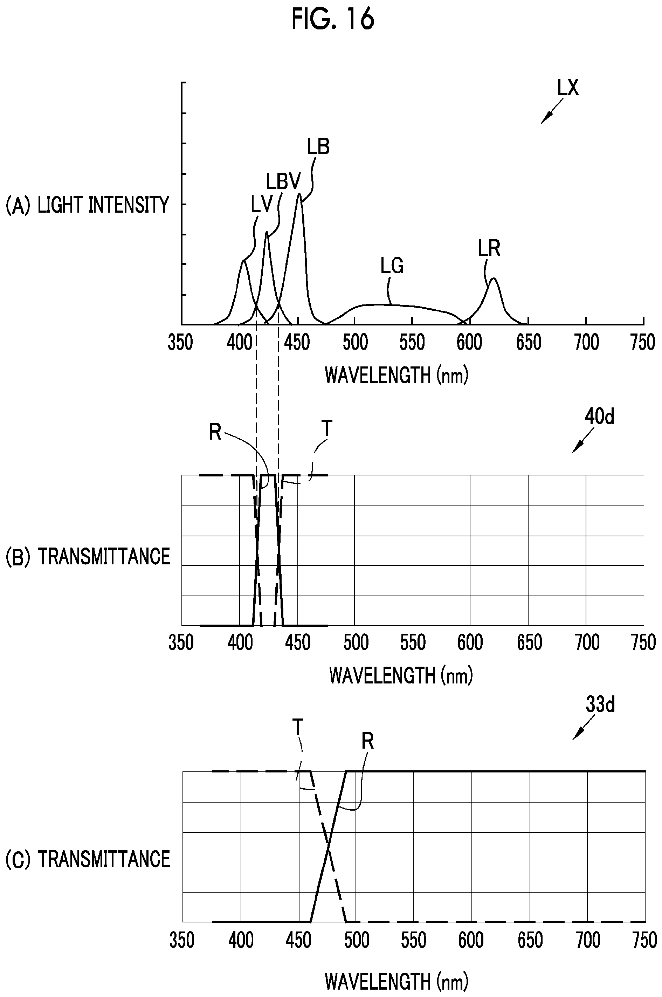

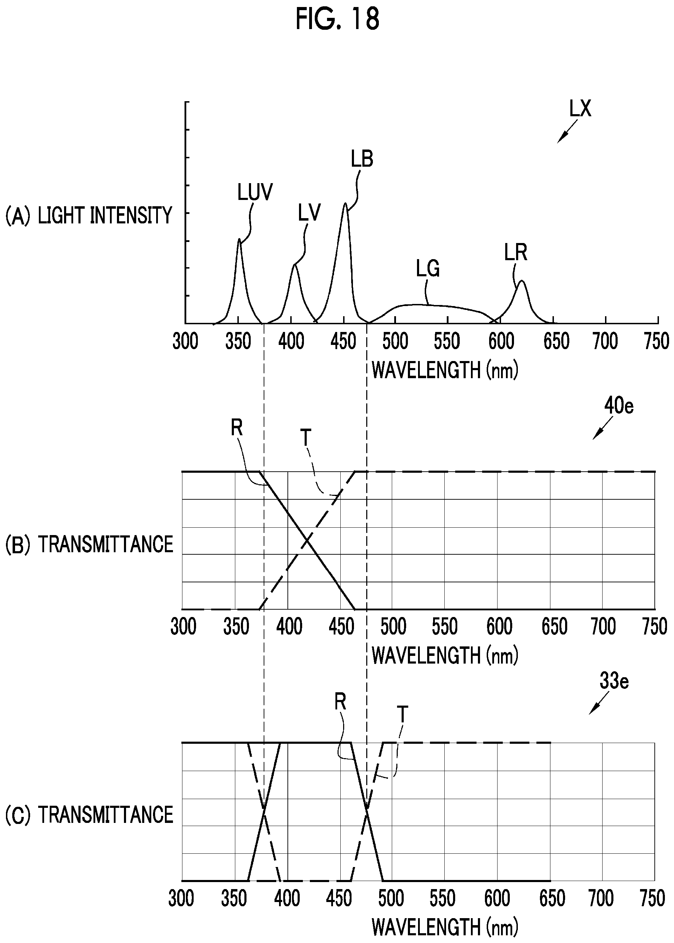

Fig. 1 is an explanatory diagram showing a configuration of an endoscope light source device including a light source attachment unit.
(A) of Fig. 2 is a graph showing spectral spectra of light emitted from a first semiconductor light source. (B) of Fig. 2 is a graph showing characteristic data of a third dichroic filter.
Fig. 3 is an explanatory diagram showing a configuration of an endoscope light source device provided with a light source attachment unit such that light emitted from a second semiconductor light source is multiplexed with an optical path of blue light.
Fig. 4 is an explanatory diagram showing a configuration of an endoscope light source device including the first semiconductor light source and the second semiconductor light source.
(A) of Fig. 5 is a graph showing spectral spectra of light emitted from the first semiconductor light source and the second semiconductor light source in a case where the second semiconductor light source emits light blue light. (B) of Fig. 5 is a graph showing characteristic data of a fourth dichroic filter. (C) of Fig. 5 is a graph showing characteristic
   data of the third dichroic filter.

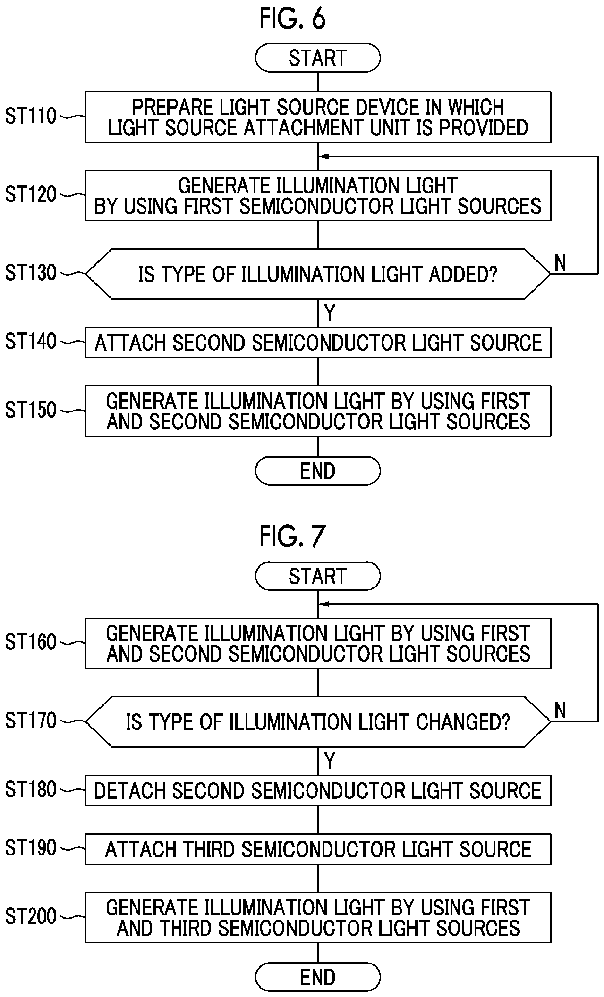

Fig. 6 is a flowchart showing an example of a procedure in a method of adding illumination light.
Fig. 7 is a flowchart showing an example of a procedure in a method of changing illumination light.

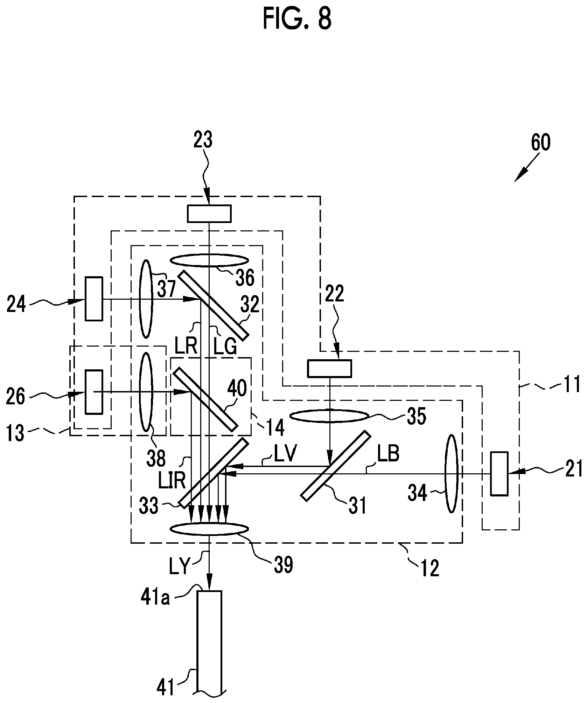

Fig. 8 is an explanatory diagram showing a configuration of an endoscope light source device including the first semiconductor light source and the third semiconductor light source.
(A) of Fig. 9 is a graph showing spectral spectra of light emitted from the first semiconductor light source and the third semiconductor light source in a case where the third semiconductor light source emits near-infrared light. (B) of Fig. 9 is a graph showing characteristic data of the fourth dichroic filter. (C) of Fig. 9 is a graph showing characteristic data of the third dichroic filter.

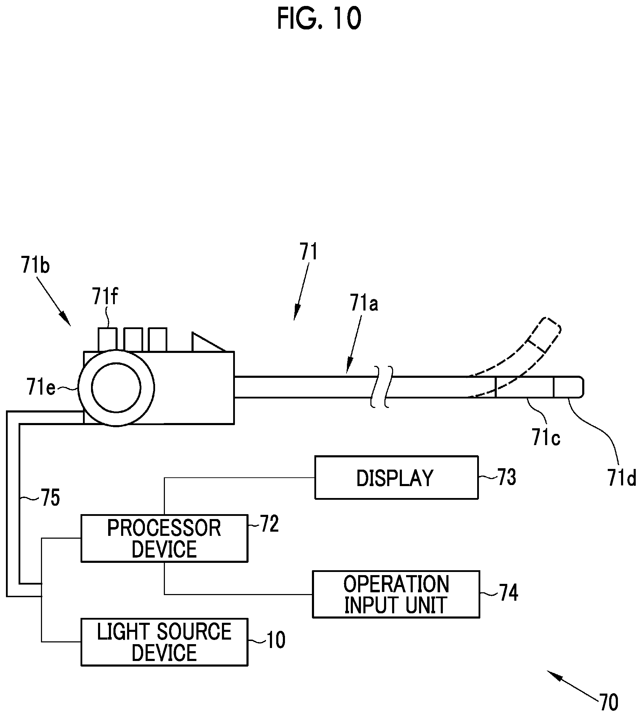

Fig. 10 is a schematic diagram showing a configuration of an endoscope system.

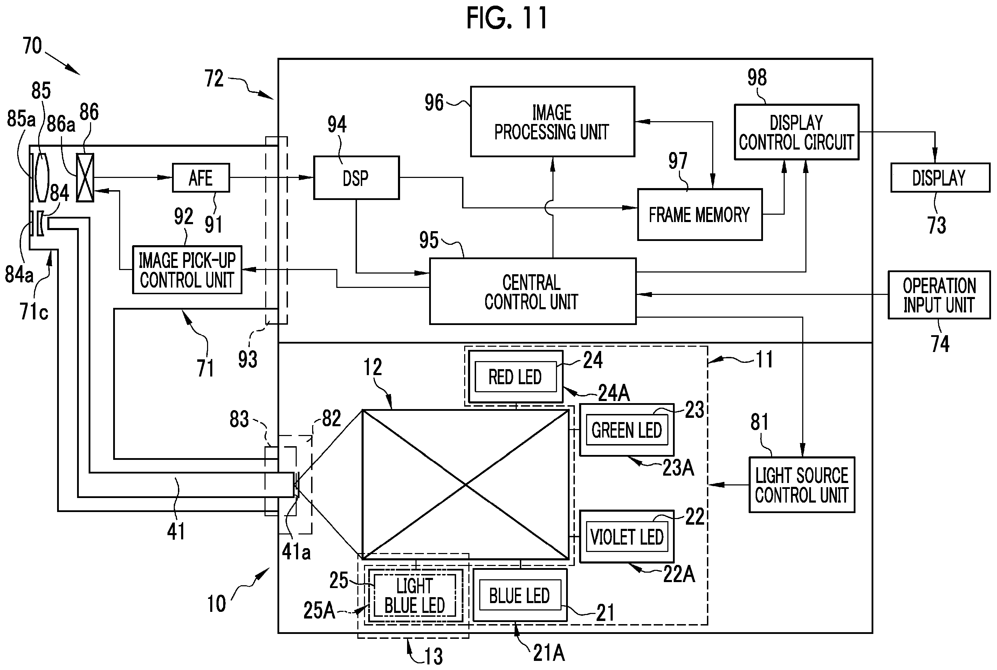

Fig. 11 is a block diagram showing a configuration of the endoscope system.

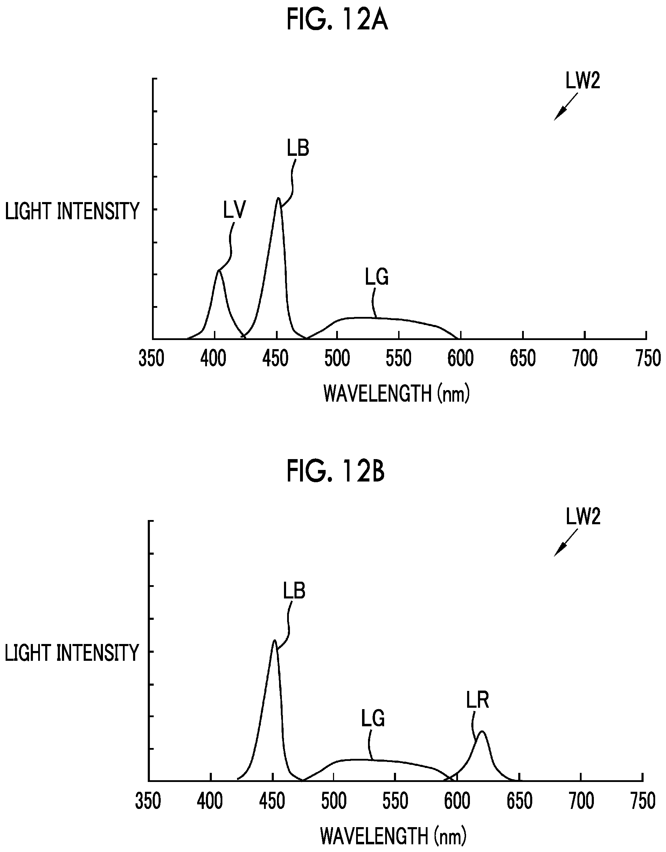

Fig. 12A is a graph showing spectral spectra of light emitted from first semiconductor light sources of three colors of a first group. Fig. 12B is a graph showing spectral spectra of light emitted from first semiconductor light sources of three colors of a second group.

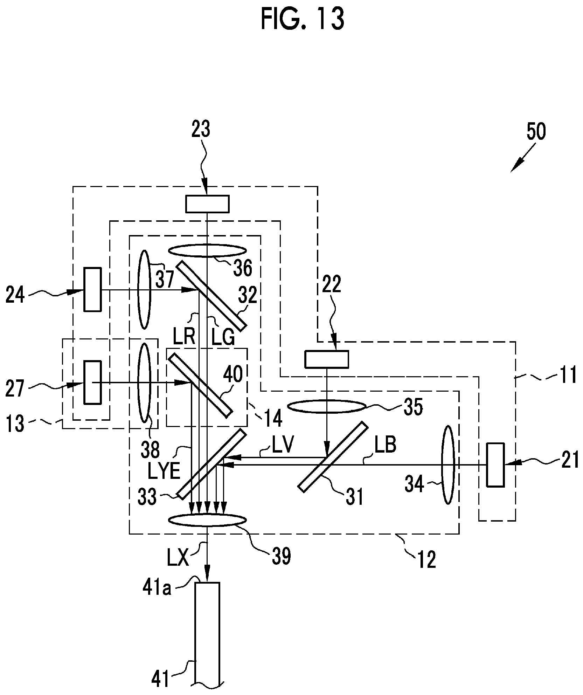

Fig. 13 is an explanatory diagram showing a configuration of an endoscope light source device including a first semiconductor light source and a yellow LED that is a second semiconductor light source.
(A) of Fig. 14 is a graph showing spectral spectra of light emitted from the first semiconductor light source and the second semiconductor light source in a case where the second semiconductor light source emits yellow light. (B) of Fig. 14 is a graph showing characteristic data of the fourth dichroic filter. (C) of Fig. 14 is a graph showing characteristic data of the third dichroic filter.

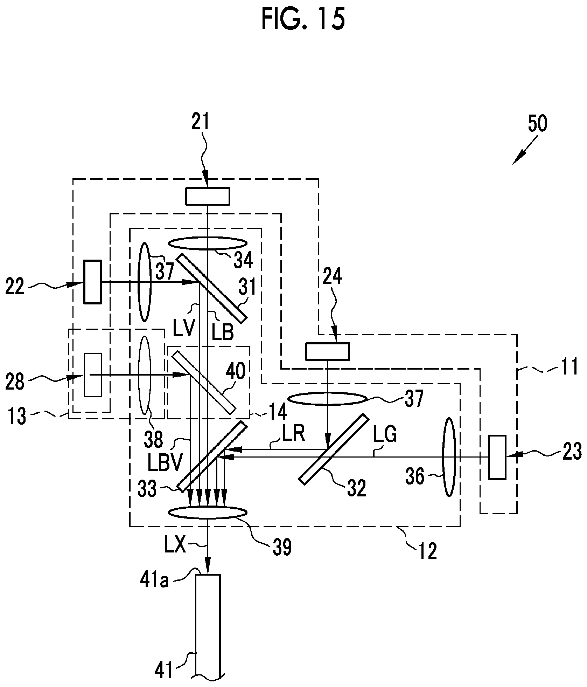

Fig. 15 is an explanatory diagram showing a configuration of an endoscope light source device including a first semiconductor light source and a blue-violet LED which is a second semiconductor light source.
(A) of Fig. 16 is a graph showing spectral spectra of light emitted from the first semiconductor light source and the second semiconductor light source in a case where the second semiconductor light source emits blue-violet light. (B) of Fig. 16 is a graph showing characteristic data of the fourth dichroic filter. (C) of Fig. 16 is a graph showing characteristic data of the third dichroic filter.

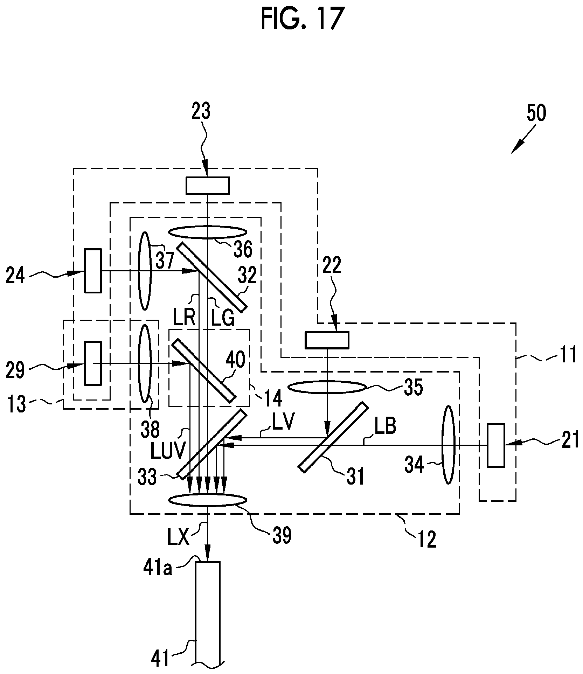

Fig. 17 is an explanatory diagram showing a configuration of an endoscope light source device including a first semiconductor light source and an ultraviolet light LED which is a second semiconductor light source.
(A) of Fig. 18 is a graph showing spectral spectra of light emitted from the first semiconductor light source and the second semiconductor light source in a case where the second semiconductor light source emits ultraviolet light. (B) of Fig. 18 is a graph showing characteristic data of the fourth dichroic filter. (C) of Fig. 18 is a graph showing characteristic data of the third dichroic filter.

### DESCRIPTION OF THE PREFERRED EMBODIMENTS

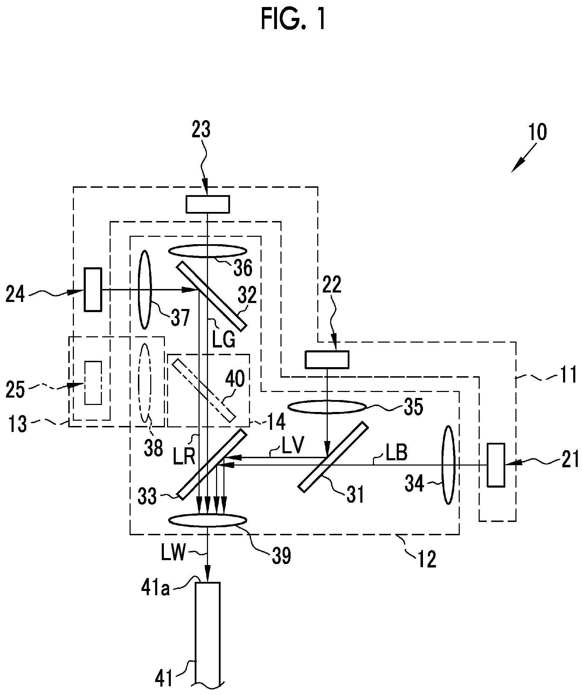

An example of a basic configuration of the present invention will be described. As shown in Fig. 1, an endoscope light source device (hereinafter, referred to as a light source device) 10 according to an embodiment of the present invention includes a light source unit 11, an illumination light generation unit 12, and a light source attachment unit 13. The light source unit 11 includes a plurality of semiconductor light sources that emit light in wavelength ranges different from each other. The plurality of semiconductor light sources are fixed to the light source device 10 and are not attachable and detachable, and these are used as first semiconductor light sources. Illumination light that is white light LW1 is generated by light emitted from each of the plurality of first semiconductor light sources.

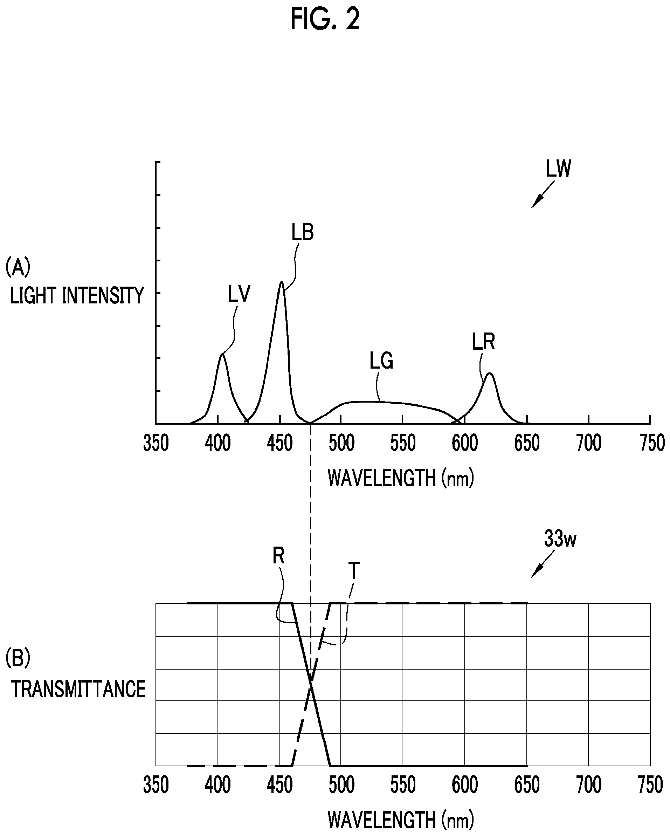

In the present embodiment, a light emitting diode (LED) is used as a semiconductor light source. The first semiconductor light sources consist of a blue LED 21 that emits blue light LB, a violet LED 22 that emits violet light LV, a green LED 23 that emits green light LG, and a red LED 24 that emits red light LR. The light source unit 11 emits, as illumination light, multicolor spectral light having a multicolor spectrum in which the violet light LV, the blue light LB, the green light LG, and the red light LR are superimposed by a plurality of light sources that independently emit light of different colors from each other. As shown in (A) of Fig. 2, the light source device 10 emits the blue light LB, the violet light LV, the green light LG, and the red light LR from the respective first semiconductor light sources. Since an amount of light emitted from each of the LEDs 21 to 24 can be controlled independently, a spectral spectrum of the multicolor spectral light can be changed by changing an amount of light from each of the LEDs 21 to 24.

The light source attachment unit 13 is configured to allow a semiconductor light source to be attached thereto and detached therefrom. A semiconductor light source attached to the light source attachment unit 13 is a second semiconductor light source. The light source unit 11 includes a plurality of first semiconductor light sources in a case where the second semiconductor light source is not attached to the light source attachment unit 13, and includes a plurality of first semiconductor light sources and a second semiconductor light source in a case where the second semiconductor light source is attached to the light source attachment unit 13. In a case where the first semiconductor light source, the second semiconductor light source, and the like are not distinguished, these will be referred to as semiconductor light sources.

At least one light source attachment unit 13 is provided in the light source device 10, and the second semiconductor light source can be attached. The attached second semiconductor light source may be detached from the light source attachment unit 13 to return to an original state, or the attached second semiconductor light source is detached and a third semiconductor light source different from the second semiconductor light source may be attached, that is, the second semiconductor light source may be changed to the third semiconductor light source. The light source attachment unit 13 includes a space for attaching the second semiconductor light source before attaching the second semiconductor light source or after detaching the attached second semiconductor light source. A plurality of light source attachment units 13 may be provided in the light source device 10. In the present embodiment, one light source attachment unit 13 is provided, and one second semiconductor light source can be attached to the light source attachment unit 13, and one attached second semiconductor light source can be detached from the light source attachment unit 13 to return to an original state.

It is assumed that, in the second semiconductor light source, a wavelength range or the like of emitted light is selected in accordance with target illumination light. For example, the second semiconductor light source is a light blue LED 25 that emits light blue light LSB.

The illumination light generation unit 12 generates white light LW1 as illumination light by multiplexing light emitted from each of the first semiconductor light sources. The illumination light generation unit 12 includes a multiplexing member, a collimator lens, and a condenser lens 39. In the multiplexing member, a multiplexing member that multiplexes the light emitted from each of the first semiconductor light sources is a first multiplexing member. The multiplexing member consists of at least a first multiplexing member in a case where the second semiconductor light source is not attached to the light source attachment unit 13, and consists of a first multiplexing member and a second multiplexing member in a case where the second semiconductor light source is attached to the light source attachment unit 13.

The multiplexing member is an optical element having a wavelength selection function. The wavelength selection function is a function of reflecting light in a specific wavelength range and transmitting light in a specific wavelength range in a case where light is incident to the multiplexing member. Even in a case where the plurality of respective semiconductor light sources emit light in different directions due to the wavelength selection function of each multiplexing member and disposition of each multiplexing member, these pieces of light are multiplexed and incident to the condenser lens 39 to generate illumination light.

In the present embodiment, the multiplexing member is a dichroic filter. The first multiplexing member consists of a first dichroic filter 31, a second dichroic filter 32, and a third dichroic filter 33. Each of the dichroic filters has a characteristic of transmitting light in a specific wavelength range and reflecting light in a specific wavelength range such that light emitted from each of the plurality of first semiconductor light sources is incident to the condenser lens 39 to generate illumination light. Characteristics of each of the multiplexing members are determined depending on disposition of each of the plurality of semiconductor light sources in the light source device 10, a wavelength range of emitted light, and the like.

The first dichroic filter 31 transmits the blue light LB and reflects the violet light LV. The second dichroic filter 32 transmits the green light LG and reflects the red light LR. The third dichroic filter 33 transmits the green light LG and the red light LR, and reflects the blue light LB and the violet light LV. As shown in (B) of Fig. 2, characteristic data 33w of the third dichroic filter 33 in this case is a characteristic in which light in a wavelength range equal to or more than the green light LG is transmitted as shown in a transmission curve T, and light in a wavelength range equal to or less than the blue light LB is reflected as shown in a reflection curve R.

It is preferable that at least one first multiplexing member is replaceable. In the present embodiment, in a case where the second semiconductor light source is attached to the light source attachment unit 13, the third dichroic filter 33 needs to transmit light emitted from the second semiconductor light source in some cases. Therefore, in a case where the third dichroic filter 33 does not transmit light emitted from the second semiconductor light source in a case where the second semiconductor light source is attached to the light source attachment unit 13, the third dichroic filter 33 is replaced with a dichroic filter having a characteristic of transmitting light emitted from the second semiconductor light source in addition to the above-described characteristics for light emitted from the first semiconductor light source.

As described above, by making at least one first multiplexing member replaceable, it is possible to increase the degree of freedom in disposition of the semiconductor light source. In a case where a wavelength range of light emitted from the second semiconductor light source to be attached to the light source attachment unit 13 is determined in advance, the third dichroic filter 33 may have in advance a characteristic of transmitting light emitted from the second semiconductor light source in addition to the above-described characteristics for light emitted from the first semiconductor light source.

The collimator lens adjusts the light emitted from each semiconductor light source to parallel light. In the present embodiment, the collimator lens includes a first collimator lens 34 to which the blue light LB emitted from the blue LED 21 is incident, a second collimator lens 35 to which the violet light LV emitted from the violet LED 22 is incident, a third collimator lens 36 to which the green light LG emitted from the green LED 23 is incident, and a fourth collimator lens 37 to which the red light LR emitted from the red LED 24 is incident.

Light emitted from each of the plurality of first semiconductor light sources is incident to the condenser lens 39 by the plurality of first semiconductor light sources included in the light source unit 11, the plurality of first multiplexing members and the plurality of collimator lenses included in the illumination light generation unit 12. The condenser lens 39 generates illumination light that is the white light LW1 by using incident light emitted from the first semiconductor light sources and emits the illumination light. Therefore, the illumination light generation unit 12 generates the illumination light that is the white light LW1 by using the light emitted from the plurality of first semiconductor light sources and supplies the illumination light to an incident end 41a of a light guide 41 included in the endoscope.

In the present embodiment, since the light emitted from the respective first semiconductor light sources is the blue light LB, the violet light LV, the green light LG, and the red light LR, the illumination light generation unit 12 generates illumination light that is the white light LW1 by using the light and supplies the illumination light to the incident end 41a of the light guide 41 included in the endoscope.

A case where the second semiconductor light source is attached to the light source attachment unit 13 will be described. In this case, illumination light is generated by light emitted from the plurality of first semiconductor light sources and light emitted from the second semiconductor light source. The illumination light generation unit 12 includes a second multiplexing member disposition portion 14 for disposing a second multiplexing member. The second multiplexing member disposition portion 14 is configured such that the second multiplexing member is attachable thereto and detachable therefrom. In the second multiplexing member disposition portion 14, the second multiplexing member for multiplexing light emitted from at least one of the plurality of first semiconductor light sources and light emitted from the second semiconductor light source is disposed.

The light source attachment unit 13 is preferably provided at a position where an optical path length of the light emitted from the second semiconductor light source to the light guide is shorter than an optical path length of the light emitted from each of the plurality of first semiconductor light sources to the light guide. That is, it is preferable to attach the light source attachment unit 13 at a position where the light emitted from the second semiconductor light source is incident last among all pieces of light from the semiconductor light sources in the order in which light from the respective semiconductor light sources is incident to the condenser lens 39. Consequently, it is possible to further suppress the influence of the light emitted from the second semiconductor light source on the other light.

The light source attachment unit 13 is preferably provided at a position where the light emitted from the second semiconductor light source is multiplexed with the light emitted from the first semiconductor light source on an optical path of the blue light LB or an optical path of the green light LG included in the plurality of first semiconductor light sources. Therefore, in this case, the plurality of first semiconductor light sources include the blue LED 21 that emits the blue light LB or the green LED 23 that emits the green light LG. It is preferable that a wavelength range of the light emitted from the second semiconductor light source exists between a wavelength range of the blue light LB and a wavelength range of the green light LG emitted from the first semiconductor light sources. In this case, the characteristic of the third dichroic filter 33 is that a cutoff wavelength is provided in a wavelength range before and after the wavelength range of the light emitted from the second semiconductor light source. For example, as the second semiconductor light source, a light blue LED 25 that emits light blue light LSB may be employed. Consequently, it is possible to prevent the characteristics of the third dichroic filter 33 from being complicated, and to suppress deterioration in basic performance of the light source device 10 such as generation of illumination light that is the white light LW1 using the first semiconductor light sources, or to suppress cost increase.

The optical path length of the blue light LB or the green light LG is set to an optical path length at which the light emitted from the second semiconductor light source can be multiplexed, and a space in which the light emitted from the second semiconductor light source can be multiplexed may be provided in the middle of the optical path of the blue light LB or the green light LG. The light source attachment unit 13 may be provided at a position where the light emitted from the second semiconductor light source can be multiplexed in the middle of the optical path of the blue light LB or the green light LG.

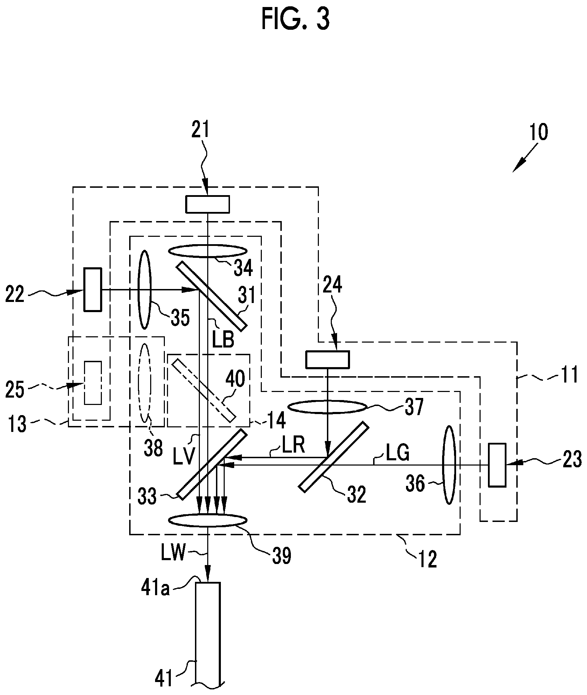

As shown in Fig. 3, in a case where the light emitted from the second semiconductor light source is multiplexed on the optical path of the blue light LB, the second multiplexing member disposition portion 14 is disposed near the optical path of the blue light LB such that the light emitted from the second semiconductor light source is multiplexed. In this case, for example, the third dichroic filter 33 reflects the green light LG and the red light LR and transmits the blue light LB and the violet light LV such that illumination light that is the white light LW1 is appropriately generated by the plurality of first semiconductor light sources. In the drawings, those having the same reference numeral indicate those having the same name.

The second multiplexing member disposition portion 14 is preferably provided at a position where the light emitted from the second semiconductor light source is multiplexed with the light from emitted from the first semiconductor light sources on the optical path of the blue light LB or the optical path of the green light LG included in the plurality of first semiconductor light sources, depending on a position of the light source attachment unit 13.

Characteristics of the second multiplexing member disposed in the second multiplexing member disposition portion 14 depend on disposition of each of the plurality of first semiconductor light sources and the second semiconductor light sources and/or a wavelength range of the light emitted from each of the semiconductor light sources. The second multiplexing member has a characteristic of reflecting the light emitted from the second semiconductor light source depending on disposition or the like of each semiconductor light source, and thus multiplexes the light emitted from at least one of the plurality of first semiconductor light sources with the light emitted from the second semiconductor light source. The second multiplexing member preferably has a characteristic of transmitting light emitted from at least one of the plurality of first semiconductor light sources. That is, the second multiplexing member preferably has a characteristic of transmitting light emitted from at least one of the plurality of first semiconductor light sources and reflecting light emitted from the second semiconductor light source.

In the present embodiment, the second multiplexing member is a fourth dichroic filter 40. The fourth dichroic filter 40 has a characteristic of transmitting light in a specific wavelength range and reflecting light in a specific wavelength range. The fourth dichroic filter 40 multiplexes light emitted from at least one of the plurality of first semiconductor light sources with light emitted from the second semiconductor light source, and causes the multiplexed light to be incident to the condenser lens 39 to generate illumination light. As described above, in a case where the second semiconductor light source is attached to the light source attachment unit 13, the third dichroic filter 33 transmits the light emitted from the second semiconductor light source.

In a case where the second semiconductor light source is attached, it is preferable to dispose a fifth collimator lens 38 for the light emitted from the second semiconductor light source. Therefore, in a case where the second semiconductor light source is attached, the illumination light generation unit 12 includes the fifth collimator lens 38 to which the light emitted from the second semiconductor light source is incident. After the second semiconductor light source is attached, the light emitted from the plurality of first semiconductor light sources and the second semiconductor light source is multiplexed by the multiplexing member to be illumination light, and the illumination light is supplied to the incident end 41a of the light guide 41 by the condenser lens 39.

In the present embodiment, in a case where the light blue LED 25 as the second semiconductor light source is attached to the light source attachment unit 13, the fourth dichroic filter 40 transmits the green light LG emitted from the green LED 23, the red light LR emitted from the red LED 24, and the light blue light LSB emitted from the light blue LED 25, reflects light in the wavelength range of the blue light LB emitted from the blue LED 21 and light in the wavelength range of the violet light LV emitted from the violet LED 22 to be incident to the condenser lens 39 (refer to (B) of Fig. 5). The light emitted from the condenser lens 39 may be illumination light having a multicolor spectrum different from illumination light having a multicolor spectrum generated by the plurality of first semiconductor light sources in a case where the second semiconductor light source is not attached. The generated illumination light is supplied to the incident end 41a of the light guide 41.

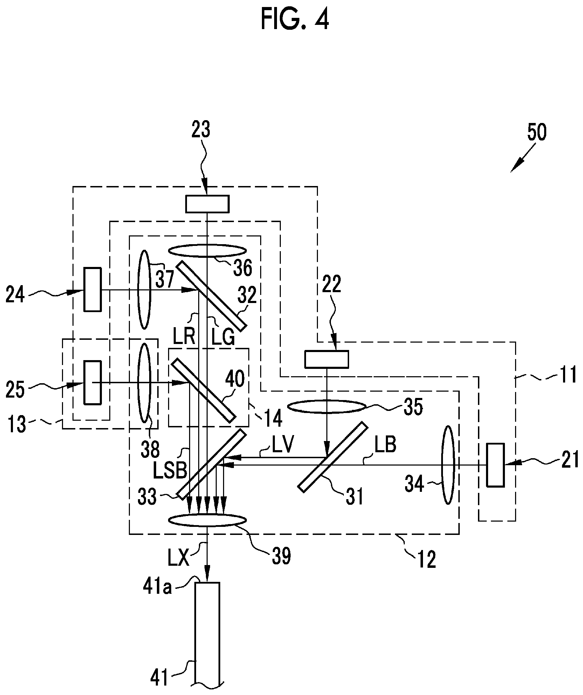

As shown in Fig. 4, a light source device 50 according to an embodiment of the present invention is a device in which the second semiconductor light source is attached to the light source attachment unit 13 in the light source device 10. The light source device 50 includes a light source unit 11, an illumination light generation unit 12, and a light source attachment unit 13 to which the second semiconductor light source is attached. The light source unit 11 includes a plurality of semiconductor light sources that emit light in wavelength ranges different from each other. The plurality of semiconductor light sources consist of a plurality of first semiconductor light sources that are not attachable and detachable, and a second semiconductor light source that is attachable and detachable. In the present embodiment, the light source device 50 includes one light source attachment unit 13, and one second semiconductor light source is attached to the light source attachment unit 13.

The light source unit 11 includes a plurality of first semiconductor light sources, and in the present embodiment, the plurality of first semiconductor light sources include a blue LED 21 that emits blue light LB, a violet LED 22 that emits violet light LV, a green LED 23 that emits green light LG, and a red LED 24 that emits red light LR. The second semiconductor light source may be a semiconductor light source that emits light in a wavelength range selected according to target illumination light to be generated. In the present embodiment, the second semiconductor light source is a light blue LED 25 that emits light blue light LSB.

As shown in (A) of Fig. 5, the light source device 50 generates illumination light LX having a specific multicolor spectrum generated by using light emitted from each of the blue LED 21, the violet LED 22, the green LED 23, and the red LED 24, which are the first semiconductor light sources, and the light blue LED 25 that is the second semiconductor light source, and supplies the Illumination light LX to the light guide 41. The light having the multicolor spectrum shown in (A) of Fig. 5 is an example of the illumination light LX, and the Illumination light LX having various multicolor spectra can be generated by adjusting, for example a wavelength range, a spectral spectrum, or an amount of light emitted from each first semiconductor light source or the second semiconductor light source.

The illumination light generation unit 12 generates illumination light by using light emitted from a plurality of semiconductor light sources. That is, the illumination light LX is generated by multiplexing the light of each color emitted from each first semiconductor light source with the light emitted from the second semiconductor light source. The illumination light generation unit 12 includes a multiplexing member, a collimator lens, and a condenser lens 39. The multiplexing member multiplexes the light emitted from each of the plurality of semiconductor light sources. The multiplexing member includes a first multiplexing member and a second multiplexing member.

Each of the first multiplexing member and the second multiplexing member has a characteristic of transmitting light in a specific wavelength range and reflecting light in a specific wavelength range such that the light emitted from each of the plurality of first semiconductor light sources and the light emitted from the second semiconductor light source are incident to the condenser lens 39 to generate illumination light. Characteristics of each of the multiplexing members are determined depending on disposition of each of the plurality of semiconductor light sources in the light source device 50, a wavelength range of emitted light, and the like.

The first multiplexing member multiplexes light emitted from the plurality of first semiconductor light sources. The second multiplexing member has a characteristic of reflecting the light emitted from the second semiconductor light source depending on disposition or the like of each semiconductor light source, and thus multiplexes the light emitted from at least one of the plurality of first semiconductor light sources with the light emitted from the second semiconductor light source. The second multiplexing member preferably has a characteristic of transmitting light emitted from at least one of the plurality of first semiconductor light sources.

The first multiplexing member consists of a first dichroic filter 31, a second dichroic filter 32, and a third dichroic filter 33, and the second multiplexing member consists of a fourth dichroic filter 40. The fourth dichroic filter 40 is disposed in the second multiplexing member disposition portion 14.

The first dichroic filter 31 and the second dichroic filter 32 have the same characteristics as described in the light source device 10. The fourth dichroic filter transmits the green light LG emitted from the green LED 23 and the red light LR emitted from the red LED 24, and reflects the light blue light LSB emitted from the light blue LED 25 of the second semiconductor light source.

As shown in (B) of Fig. 5, characteristic data 40a of the fourth dichroic filter 40 in this case is a characteristic in which light in a wavelength range equal to or more than the green light LG is transmitted as shown in a transmission curve T, and light in a wavelength range of the light blue light LSB is reflected as shown in a reflection curve R. That is, it is assumed that a cutoff wavelength of the fourth dichroic filter 40 is on a longer wavelength side than the wavelength range of the light blue light LSB and on a shorter wavelength side than the wavelength range of the green light LG.

The third dichroic filter 33 transmits the green light LG emitted from the green LED 23, the red light LR emitted from the red LED 24, and the light blue light LSB emitted from the light blue LED 25, and reflects the blue light LB emitted from the blue LED 21 and the violet light LV emitted from the violet LED 22.

As shown in (C) of Fig. 5, characteristic data 33a of the third dichroic filter 33 in this case is a characteristic in which light in a wavelength range equal to or more than the light blue light LSB is transmitted as shown in a transmission curve T, and light in which a wavelength range equal to or less than the blue light LB is reflected as shown in a reflection curve R. That is, it is assumed that a cutoff wavelength of the third dichroic filter 33 is on a longer wavelength side than the wavelength range of the blue light LB and on a shorter wavelength side than the wavelength range of the light blue light LSB.

The collimator lens is provided in each of the plurality of semiconductor light sources. In the present embodiment, the collimator lenses include a first collimator lens 34 to which the blue light LB emitted from the blue LED 21 is incident, a second collimator lens 35 to which the violet light LV emitted from the violet LED 22 is incident, a third collimator lens 36 to which the green light LG emitted from the green LED 23 is incident, a fourth collimator lens 37 to which the red light LR emitted from the red LED 24 is incident, and a fifth collimator lens 38 to which the light blue light LSB emitted from the light blue LED 25 is incident.

Light emitted from the plurality of first semiconductor light sources and the second semiconductor light source is incident to the condenser lens 39 by the plurality of semiconductor light sources included in the light source unit 11, the first multiplexing member and the second multiplexing member included in the illumination light generation unit 12, and the plurality of collimator lenses. The condenser lens 39 emits the illumination light LX generated by using incident light emitted from the plurality of semiconductor light sources. Regarding the illumination light LX thus generated, the type of illumination light having a multicolor spectrum, a spectral spectrum, or an amount of light, or the like is controlled according to, for example, a wavelength range and an amount of light emitted from each of the plurality of first semiconductor light sources and the second semiconductor light source. The generated illumination light LX is supplied to the incident end 41a of the light guide 41 included in the endoscope.

In a case where the attachable and detachable second semiconductor light source is detached, the light source device 50 is the light source device 10 (refer to Fig. 1) and generates illumination light that is the white light LW1 by using the light emitted from the plurality of first semiconductor light sources. The light source attachment unit 13 includes a space after the second semiconductor light source is detached. The second multiplexing member disposition portion 14 is configured such that the second multiplexing member is attachable thereto and detachable therefrom. Therefore, the fourth dichroic filter 40, which is the second multiplexing member, disposed in a case where the second semiconductor light source is attached, is preferably detached in a case where the second semiconductor light source is detached. The third dichroic filter 33 adjusted to transmit the light emitted from the second semiconductor light source may be replaced with a dichroic filter that transmits only the light emitted from the plurality of first semiconductor light sources.

Since the light source attachment unit 13 is configured such that a semiconductor light source is attachable and detachable, the light source attachment unit 13 may be left as it is after the second semiconductor light source is detached, or third semiconductor light source that emits light in a wavelength range different from that of each of the first semiconductor light sources and the second semiconductor light source may be attached, or the second semiconductor light source may be attached again. In a case where the third semiconductor light source is attached, a dichroic filter for the third semiconductor light source is disposed in the second multiplexing member disposition portion 14 in the same manner as in the case where the second semiconductor light source is attached. In some cases, the third dichroic filter 33 is replaced with a dichroic filter that transmits light emitted from the third semiconductor light source.

A method of adding illumination light in the light source device according to an embodiment of the present invention is a method of adding illumination light in the light source device that supplies the illumination light to the light guide of the endoscope. The addition of the illumination light is to add the type of the illumination light supplied by the light source device, and the type of the illumination light is distinguished by a spectrum of the illumination light, which is a multicolor spectrum. A target device to which the illumination light is added is a device in which a light source is not attached to the light source attachment unit 13, and is the light source device 10 (refer to Fig. 1 or Fig. 3) in the present embodiment. According to the method of adding illumination light in the embodiment of the present invention, it is possible to add the type of illumination light different from the type of illumination light previously supplied by the light source device 10 as the type of illumination light supplied by the light source device 10.

The light source device 10 includes the plurality of first semiconductor light sources, the illumination light generation unit 12, and the light source attachment unit 13. In the light source device 10, at least white light LW1 is generated as illumination light by the plurality of first semiconductor light sources and the illumination light generation unit 12.

An example of a method of adding illumination light in the light source device according to the embodiment of the present invention will be described with reference to the flowchart of Fig. 6. First, the light source device 10 (refer to Fig. 1) provided with the light source attachment unit 13 is prepared (step ST110). At least one light source attachment unit 13 is provided in the light source device 10, and is configured such that the second semiconductor light source is attachable and detachable. The light source attachment unit 13 includes a space in the light source device 10 for attaching the second semiconductor light source to the light source device 10. In a case where the light source attachment unit 13 is provided in the light source device 10, it is not necessary to provide the light source attachment unit 13 after the light source device 10 is completed. By completing the light source device 10 in which the light source attachment unit 13 is provided after providing the light source attachment unit 13 and the like at an appropriate position or the like of the light source device 10 during manufacturing of the light source device 10, the light source attachment unit 13 can be easily provided in the light source device 10. In the light source device 10 provided with the light source attachment unit 13, at least white light LW1 is generated as illumination light by the plurality of first semiconductor light sources and the illumination light generation unit 12 (step ST120).

Next, in a case where the type of illumination light is added in the light source device 10 (Y in step ST130), the second semiconductor light source is attached to the light source attachment unit 13 (step ST140). In this case, a multiplexing member for the second semiconductor light source, a collimator lens, and the like are disposed such that light emitted from the plurality of first semiconductor light sources and the attached second semiconductor light source can be multiplexed to generate the illumination light. Depending on disposition or the like of each semiconductor light source, in a case where a multiplexing member provided immediately before the condenser lens 39, that is, a multiplexing member to which light emitted from the first semiconductor light source and the second semiconductor light source is incident does not have a characteristic of transmitting light emitted from the second semiconductor light source before the second semiconductor light source is attached, this multiplexing member is replaced with a multiplexing member having the characteristic of transmitting light emitted from the second semiconductor light source.

The device after the second semiconductor light source or the like is attached to the light source device 10 is the light source device 50 (Fig. 4). Illumination light is generated by the light source device 50 to which the second semiconductor light source or the like is attached (step ST150). In a case where the type of illumination light is not added in the light source device 10 (N in step ST130), the light source device 10 subsequently generates illumination light that is at least white light LW1 by using the first semiconductor light source (step ST120).

The light emitted from the second semiconductor light source has a wavelength range or an amount of light in which illumination light having a multicolor spectrum that cannot be generated only by the plurality of first semiconductor light sources can be generated by using the light emitted from the plurality of first semiconductor light sources and the light emitted from the second semiconductor light source. Therefore, in the light source device 50, the illumination light generation unit 12 can additionally generate the type of illumination light that cannot be generated only by the plurality of first semiconductor light sources, by using the light emitted from the plurality of first semiconductor light sources and the light emitted from the second semiconductor light source. That is, the illumination light generation unit 12 generates the illumination light LX different from illumination light that is white light generated by using the light emitted from at least the plurality of first semiconductor light sources, by using the light emitted from the plurality of first semiconductor light sources and the light emitted from the second semiconductor light source.

As described above, according to the method of adding illumination light in the light source device related to the embodiment of the present invention, in the light source device 10, in addition to the type of illumination light generated by the light emitted from the plurality of first semiconductor light sources, by multiplexing the light emitted from the plurality of first semiconductor light sources with the light emitted from the second semiconductor light source, it is possible to additionally generate the type of illumination light that cannot be realized before the second semiconductor light source is attached.

A method of changing the illumination light in the light source device according to the embodiment of the present invention is a method of changing illumination light in the light source device that supplies the illumination light to the light guide of the endoscope. The change of the illumination light is to change the type of illumination light supplied by the light source device, and the type of the illumination light is distinguished by a spectrum of the illumination light, which is a multicolor spectrum. A target device for changing illumination light is a device in which a light source is attached to the light source attachment unit 13, and is the light source device 50 (Fig. 4) in the present embodiment. According to the method of changing illumination light related to the embodiment of the present invention, it is possible to change the type of illumination light to be supplied from the type of illumination light previously supplied by the light source device 50 to another type of illumination light.

The light source device 50 includes the plurality of semiconductor light sources and the illumination light generation unit 12. The plurality of semiconductor light sources consist of a plurality of first semiconductor light sources that are not attachable and detachable, and a second semiconductor light source that is attachable and detachable, and emit light in wavelength ranges different from each other. The second semiconductor light source is attached to at least one light source attachment unit 13 provided in the light source devices 50. The illumination light generation unit 12 generates a plurality of types of illumination light LX by using light emitted from a plurality of semiconductor light sources including the plurality of first semiconductor light sources and the second semiconductor light source.

An example of a method of changing illumination light in the light source device according to the embodiment of the present invention will be described with reference to a flowchart of Fig. 7. First, in the light source device 50, illumination light is generated by the plurality of first semiconductor light sources and the second semiconductor light source (step ST160). In a case where the type of illumination light generated in the light source device 50 is changed (Y in step ST170), the second semiconductor light source attached to the light source attachment unit 13 is detached (step ST180). In this case, it is preferable to detach the multiplexing member, which is disposed to multiplex light emitted from the second semiconductor light source with light emitted from the first semiconductor light sources, the collimator lens for the second semiconductor light source, and the like. The light source device 10 (refer to Fig. 1) is a device after the second semiconductor light source and the like are detached.

Next, the third semiconductor light source is attached to the light source attachment unit 13 (step ST190). In the third semiconductor light source, a wavelength range or the like of the semiconductor light source is selected in accordance with target illumination light. Light emitted from the third semiconductor light source has a wavelength range or an amount of light in which illumination light having a multicolor spectrum that cannot be generated only by the plurality of first semiconductor light sources and the second semiconductor light source can be generated by using the light emitted from the plurality of first semiconductor light sources and the light emitted from the third semiconductor light source. For example, the third semiconductor light source is a near-infrared light LED 26 that emits near-infrared light IR.

In a case where the third semiconductor light source is attached, the illumination light generation unit 12 includes a multiplexing member for the third semiconductor light source, a collimator lens, and the like disposed such that the illumination light is generated by multiplexing the light emitted from the plurality of first semiconductor light sources and the attached third semiconductor light source. In this case, the multiplexing member for the second semiconductor light source is replaced with the multiplexing member for the third semiconductor light source such that illumination light can be generated by multiplexing light emitted from the plurality of first semiconductor light sources and the third semiconductor light source changed from the second semiconductor light source.

In a case where a wavelength range of the light emitted from the third semiconductor light source is determined in advance, the multiplexing member for the second semiconductor light source may have in advance a characteristic of multiplexing the light emitted from the plurality of first semiconductor light sources with both the light emitted from the second semiconductor light source and the light emitted from the third semiconductor light source. The same applies to a multiplexing member provided immediately before the condenser lens 39, that is, a multiplexing member to which light emitted from the first semiconductor light sources and the second semiconductor light source is incident, and in a case where the multiplexing member does not have a characteristic of transmitting light emitted from the third semiconductor light source before the second semiconductor light source is changed to the third semiconductor light source, the multiplexing member may be replaced with a multiplexing member having a characteristic of transmitting light emitted from the third semiconductor light source or may have in advance a characteristic of transmitting both light emitted from the second semiconductor light source and light emitted from the third semiconductor light source.

In a case where the type of the illumination light is not changed in the light source device 50 (N in step ST170), the light source device 50 subsequently generates the illumination light LX generated by the plurality of first semiconductor light sources and the second semiconductor light source (step ST120).

As shown in Fig. 8, a light source device 60 is a device after detaching the second semiconductor light source in the light source device 50 and then attaching the near-infrared light LED 26 to the light source attachment unit 13 as the third semiconductor light source. The light source device 60 includes the near-infrared light LED 26 that is the third semiconductor light source instead of the light blue LED 25 that is the second semiconductor light source, and is a device in which the fourth dichroic filter 40 is disposed in the second multiplexing member disposition portion 14.

As shown in (A) of Fig. 9, the light source device 60 generates illumination light LY having a specific multicolor spectrum generated by using light emitted from each of the blue LED 21, the violet LED 22, the green LED 23, and the red LED 24, which are the first semiconductor light sources, and the near-infrared light LED 26 that is the third semiconductor light source, and supplies the Illumination light LY to the light guide 41. The light having the multicolor spectrum shown in (A) of Fig. 9 is an example of the illumination light LY, and the Illumination light LY having various multicolor spectra can be generated by adjusting, for example, a spectral spectrum or an amount of light emitted from each of the first semiconductor light sources or the third semiconductor light source.

The fourth dichroic filter 40 and the third dichroic filter 33 appropriately transmit or reflect the near-infrared light IR emitted from the third semiconductor light source and other light emitted from each of the plurality of first semiconductor light sources such that the illumination light LY is appropriately formed by the near-infrared light IR emitted from the third semiconductor light source and the other light emitted from each of the plurality of first semiconductor light sources.

As shown in (B) of Fig. 9, characteristic data 40b of the fourth dichroic filter 40 in this case is a characteristic in which light in a wavelength range of the light blue light LSB is reflected as shown in a reflection curve R, and light in a wavelength range equal to or less than the red light LR is transmitted as shown in a transmission curve T. That is, it is assumed that a cutoff wavelength of the fourth dichroic filter 40 is on a longer wavelength side than the wavelength range of the red light LR and on a shorter wavelength side than the wavelength range of the near-infrared light LIR.

The third dichroic filter 33 transmits the green light LG emitted from the green LED 23, the red light LR emitted from the red LED 24, and the light blue light LSB emitted from the light blue LED 25, and reflects the blue light LB emitted from the blue LED 21 and the violet light LV emitted from the violet LED 22.

As shown in (C) of Fig. 9, characteristic data 33a of the third dichroic filter 33 in this case is a characteristic in which light in a wavelength range equal to or more than the green light LG is transmitted as shown in a transmission curve T, and light in a wavelength range equal to or less than the blue light LB is reflected as shown in a reflection curve R. That is, it is assumed that a cutoff wavelength of the third dichroic filter 33 is on a longer wavelength side than the wavelength range of the blue light LB and on a shorter wavelength side than the wavelength range of the light blue light LSB. However, in this case, in order to transmit the near-infrared light IR, the third dichroic filter 33 is assumed to have a characteristic of guaranteeing the transmission of the near-infrared light IR, as necessary.

In the light source device 60, the illumination light generation unit 12 can change the illumination light LX generated by the plurality of first semiconductor light sources and the second semiconductor light source in the light source device 50 to generate the illumination light LY of a type that cannot be generated only by the plurality of first semiconductor light sources and the second semiconductor light source by using the light emitted from the plurality of first semiconductor light sources and the light emitted from the third semiconductor light source.

As described above, according to the method of changing illumination light in the light source device related to the embodiment of the present invention, in the light source device 50, the illumination light LX generated by the light emitted from each of the plurality of first semiconductor light sources and the second semiconductor light source can be changed to the illumination light LY that can be realized in a case where the third semiconductor light sources are attached by multiplexing the light emitted from the plurality of first semiconductor light sources with the light emitted from the third semiconductor light source.

There is a light source device that configures a multicolored light source by multiplexing light emitted from semiconductor light sources such as a plurality of LEDs by using a multiplexing member such as a dichroic filter. Image enhanced endoscopy (IEE) for obtaining a target image or the like by using illumination light having various spectra has been developed, and it is becoming more and more important to obtain illumination light having a desired spectrum. However, in these light source devices, in order to add or replace another LED having a different wavelength range of emitted light, it is necessary to redesign the entire optical system. Such redesign is troublesome and cannot be easily realized. Even though the redesign is performed, there is concern that the light propagation efficiency of an existing general-purpose portion that emits LED light, which is already disposed, is lowered, and thus the performance may be changed or the cost may be increased.

In the light source device 10, it is easy to add the second semiconductor light source having a different wavelength range of emitted light to the first semiconductor light source that is not attachable and detachable and is attached to the device. In the light source device 50, it is easy to replace the second semiconductor light source that is attachably and detachably attached to the device. Even in a case where the second semiconductor light source is added or replaced, it is not necessary to redesign a general-purpose portion consisting of the first semiconductor light source. By appropriately providing the light source attachment unit 13, it is possible to suppress the influence of the general-purpose portion on the performance of the first semiconductor light source to be small. In particular, it is preferable that the light source attachment unit 13 is provided at a position such as on the optical path of the green light LG or the blue light LB. The light source device 60 is a device in which a spectrum of generated illumination light is easily changed. In a case of attaching, detaching, or replacing the second semiconductor light source, the second multiplexing member or the like is attachably and detachably configured, so that illumination light can be appropriately generated even in a case where the second semiconductor light source is used. It is possible to increase the degree of freedom of light emitted from a semiconductor light source used for the second semiconductor light source to be later attached to the light source device 10 or the like or the third semiconductor light source to be changed. Since at least one first multiplexing member is replaceable, it is possible to appropriately generate illumination light even in a case where the second semiconductor light source is used.

As described above, according to the light source devices 10, 50, and 60, addition or change of a light source can be easily realized. According to the method of adding illumination light or the method of changing illumination light in the light source device related to the embodiment of the present invention, addition or change of a light source can be easily realized.

Next, an endoscope system or the like including the light source device according to the embodiment of the present invention will be described. As shown in Fig. 10, an endoscope system 70 includes an endoscope 71, a light source device 10, a processor device 72, a display 73, an operation input unit 74, and a universal cord 75.

The endoscope 71 images an observation target in a living body. The light source device 10 supplies illumination light applied to the observation target to the endoscope 71. The processor device 72 functions as a central control unit that performs control related to imaging, illumination light, or the like, and also processes an image signal obtained through imaging to generate a display image or the like. The processor device 72 is electrically connected to the display 73 and the operation input unit 74. The display 73 displays an image captured by the endoscope 71, image information incidental to the image, and the like. The operation input unit 74 functions as a user interface that receives input operations such as function settings. The universal cord 75 is a cord through which a communication cable extending from an insertion part 71a, a light guide 41, or the like is inserted, and connects the endoscope 71 to the processor device 72 and the light source device 10. Therefore, the endoscope 71 has the light guide 41 that guides illumination light. An external recording unit (not shown) for recording an image, image information, or the like may be connected to the processor device 72.

The endoscope 71 is optically connected to the light source device 10 and electrically connected to the processor device 72. The endoscope 71 has an insertion part 71a to be inserted into a subject, an operating part 71b provided at a base end portion of the insertion part 71a, and a bendable part 71c and a tip part 71d provided on a tip end side of the insertion part 71a. The bendable part 71c is bent by operating an angle knob 71e of the operating part 71b. The tip part 71d is directed in a desired direction due to the bending operation. The operating part 71b is provided with a zoom operating part 71f or the like in addition to the angle knob 71e.

As shown in Fig. 11, the light source device 10 is a device that supplies illumination light applied to an observation target to the light guide 41 of the endoscope 71, and includes a light source unit 11 having a plurality of light sources, a light source control unit 81 that controls each light source of the light source unit 11, an illumination light generation unit 12 that generates illumination light by coupling an optical path of light emitted from the light source unit 11, and a light source attachment unit 13 that is configured such that a second semiconductor light source is attachable thereto and detachable therefrom.

The light source unit 11 includes a plurality of first semiconductor light sources that independently emit light of different colors from each other. Specifically, the light source unit 11 includes a violet semiconductor light source 22A having a violet LED 22 as a light emitting element, a blue semiconductor light source 21A having a blue LED 21 as a light emitting element, a green semiconductor light source 23A having a green LED 23 as a light emitting element, and a red semiconductor light source 24A having a red LED 24 as a light emitting element.

The violet LED 22 emits violet light LV having a central wavelength of 405 nm and a wavelength range of 380 to 420 nm. The blue LED 21 emits blue light LB having a central wavelength of 460 nm and a wavelength range of 420 to 500 nm. The green LED 23 emits green light LG having a wavelength range of 480 to 600 nm. The red LED 24 emits red light LR having a central wavelength of 620 to 630 nm and a wavelength range of 600 to 650 nm (refer to Fig. 2). The central wavelengths of the violet LED 22 and the blue LED 21 have a width of about ±5 nm to ±10 nm.

The light source attachment unit 13 is configured such that a second semiconductor light source is attachable thereto and detachable therefrom. In a case where the second semiconductor light source is attached to the light source attachment unit 13, the light source unit 11 includes a plurality of first semiconductor light sources and a second semiconductor light source. The second semiconductor light source attached to the light source attachment unit 13 may be detached and a third semiconductor light source may be attached instead.

The light source control unit 81 individually controls a drive current, or a drive voltage for each of the LEDs 21 to 24 included in the light source unit 11 or a pulse width, a pulse length, or the like in a case where the drive current or the drive voltage is pulse-input to each of the LEDs 21 to 24. Consequently, the light source control unit 81 controls an emission timing or an amount of light emitted from each of the LEDs 21 to 24. Specifically, the light source control unit 81 controls each of the LEDs 21 to 24 of the light source unit 11, and superimposes the violet light LV, the blue light LB, the green light LG, and the red light LR emitted from the respective LEDs 21 to 24 to generate first multicolor spectral light. The light source control unit 81 controls an emission timing or an amount of light from each of the LEDs 21 to 24 to convert the first multicolor spectral light into white light LW (refer to Fig. 1).

The illumination light generation unit 12 integrates the pieces of color light emitted from the respective LEDs 21 to 24. A light emitting portion of the illumination light generation unit 12 is disposed in the vicinity of a receptacle connector 82 to which a light source connector 83 is connected. The illumination light generation unit 12 emits the light incident from each of the LEDs 21 to 24 to the incident end 41a of the light guide 41 of the endoscope 71.

A connector is attached to one end of the processor device 72 and the light source device 10 side. The connector is a composite type connector consisting of a communication connector 93 and the light source connector 83. The communication connector 93 and the light source connector 83 are attachably and detachably connected to the processor device 72 and the light source device 10, respectively. One end of the communication cable is disposed in the communication connector 93, and the incident end 41a of the light guide 41 is disposed in the light source connector 83.

The endoscope 71 includes a light guide 41, an image pick-up element 86, an analog front end (AFE) 91 (analog processing circuit), and an image pick-up control unit 92. The light guide 41 is a fiber bundle in which a plurality of optical fibers are bundled.

An irradiation lens 84 is disposed at the tip part 71d of the endoscope, and illumination light supplied from the light source device 10 is guided to the irradiation lens 84 by the light guide 41 and is applied from an illumination window 84a toward an observation target. An objective optical system 85 and the image pick-up element 86 are disposed behind the observation window 85a. An image of the observation target is incident to the objective optical system 85 through the observation window 85a, and is formed on an image pick-up surface 86a of the image pick-up element 86 by the objective optical system 85.

The image pick-up element 86 consists of a charge-coupled device (CCD) image sensor, a complementary metal oxide semiconductor (CMOS) image sensor, or the like, and a plurality of photoelectric conversion elements configuring pixels such as photodiodes are arranged in a matrix on the image pick-up surface 86a. The image pick-up element 86 performs photoelectric conversion of light received by the image pick-up surface 86a, and stores signal charge corresponding to an amount of light received in each pixel. The signal charge is converted into a voltage signal by an amplifier and read. The voltage signal is output from the image pick-up element 86 to the AFE 91 as an image signal.

The AFE 91 includes a correlated double sampling circuit (CDS), an automatic gain control circuit (AGC), and an analog/digital converter (A/D) (none thereof shown). The CDS performs a correlation double sampling process on the analog image signal from the image pick-up element 86 to remove noise caused by resetting the signal charge. The AGC amplifies the image signal from which noise has been removed by the CDS. The A/D converts the image signal amplified by the AGC into a digital image signal having a grayscale value corresponding to a predetermined number of bits and inputs the image signal to the processor device 72.

The image pick-up control unit 92 is connected to a central control unit 95 in the processor device 72, and inputs a drive signal to the image pick-up element 86 in synchronization with a reference clock signal input from the central control unit 95. The image pick-up element 86 outputs an image signal to the AFE 91 at a predetermined frame rate on the basis of the drive signal from the image pick-up control unit 92.

The image pick-up element 86 is a color image pick-up element, and microcolor filters of three colors of blue, green, and red are assigned to respective pixels on the image pick-up surface 86a. Arrangement of the microcolor filters is, for example, a Bayer array. The image pick-up element 86 performs a storage operation of storing the signal charge in the pixel and a reading operation of reading the stored signal charge within an acquisition period of one frame. The image pick-up element 86 sequentially outputs an image signal for one frame in which pixel values of blue, green, and red pixels are mixed in accordance with the reading timing, according to a frame rate. Such an image pick-up operation is repeatedly performed while observation using the endoscope system 70 is set.

In addition to the central control unit 95, the processor device 72 includes a digital signal processor (DSP) 94, an image processing unit 96, a frame memory 97, and a display control circuit 98. In the processor device 72, a program related to processing such as image pick-up, control of illumination light, or image processing is stored in a program memory (not shown). In the processor device 72, the central control unit 95 configured by a processor or the like operates the program in the program memory to realize various functions of the DSP 94, the central control unit 95, the image processing unit 96, the frame memory 97, and the display control circuit 98. The central control unit 95 receives information from the endoscope 71 and the light source device 10, and not only controls each unit of the processor device 72 but also controls the endoscope 71 or the light source device 10 on the basis of the received information. Information such as an instruction from the operation input unit 74 is also received.

The DSP 94 acquires an image signal output by the image pick-up element 86. The DSP 94 separates an image signal in which signals respectively corresponding to the blue, green, and red pixels are mixed into a blue image signal, a green image signal, or a red image signal, and performs a pixel interpolation process on the image signals of the respective colors. The DSP 94 performs signal processing such as gamma correction and white balance correction on each of the blue, green, or red image signals.

The DSP 94 calculates an exposure value on the basis of the blue image signal, the green image signal, and the red image signal, and outputs, to the central control unit 95, an exposure control signal for performing control such that in a case where an amount of light of the entire image is insufficient (underexposure), an amount of illumination light is increased, and, on the other hand, in a case where an amount of light is too large (overexposure), an amount of illumination light is reduced. The central control unit 95 transmits the exposure control signal to the light source control unit 81 of the light source device 10.

The frame memory 97 stores image data output by the DSP 94 or processed image data processed by the image processing unit 96. The display control circuit 98 reads the image data that has undergone image processing from the frame memory 97, converts the image data into a video signal such as a composite signal or a component signal, and outputs the image data to the display 73.

The endoscope system 70 may perform image enhanced endoscopy (IEE) using an image signal obtained by imaging an observation target illuminated with illumination light having a desired multicolor spectrum. The endoscope system 70 has a plurality of observation modes, and is set in advance to perform specific image enhanced endoscopy in each observation mode.

The light source device 50 is a device in which the second semiconductor light source is added to the light source attachment unit 13 of the light source device 10, and the light source device 60 is a device in which the third semiconductor light source is added to the light source attachment unit 13 of the light source device 10 instead of the second semiconductor light source. In a case where the light source device 10 is used, in order to perform image enhanced endoscopy using illumination light having a multicolor spectrum, which cannot be realized by the first semiconductor light sources of four colors included in the light source device 10, the second semiconductor light source that emits light that enables illumination light having a multicolor spectrum desired to be realized is attached to the light source attachment unit 13 of the light source device 10 to form the light source device 50. The light source device 50 can realize illumination light having a multicolor spectrum, which cannot be realized by the first semiconductor light sources of four colors included in the light source device 10, by adding the second semiconductor light source.

In the light source device 50, in order to perform image enhanced endoscopy using illumination light having a multicolor spectrum that cannot be realized by the plurality of first semiconductor light sources and the second semiconductor light source, the second semiconductor light source is detached from the light source attachment unit 13, and then the third semiconductor light source is attached to the light source attachment unit 13 to form the light source device 60. The light source device 60 can realize illumination light having a multicolor spectrum, which cannot be realized by the first semiconductor light sources of four colors included in the light source device 10 and the semiconductor light sources of five colors included in the light source device 50, by changing the second semiconductor light source to the third semiconductor light source.

In the light source device 50, the first semiconductor light source, the second semiconductor light source, and the third semiconductor light source may be LEDs that emit excitation light. In this case, a phosphor that emits fluorescence by applying the excitation light is disposed on an optical path. Consequently, fluorescence can be emitted in response to the excitation light emitted from these semiconductor light sources.

In a normal observation mode, observation is performed by using illumination light that is the white light LW1. The first semiconductor light sources included in the light source device 10 (refer to Fig. 1) consist of LEDs of four colors such as the blue LED 21 that emits the blue light LB, the violet LED 22 that emits the violet light LV, the green LED 23 that emits the green light LG, and the red LED 24 that emits the red light LR. The light source device 10 generates illumination light that is the white light LW1 (refer to Fig. 1) by using the light emitted from each of the first semiconductor light sources of four colors.

For example, first semiconductor light sources of at least three colors may be selected from among the semiconductor light sources of four colors, and illumination light having a continuous wavelength range may be generated by using light emitted from each of the first semiconductor light sources of three colors. As shown in Figs. 12A and 12B, in a case where the first semiconductor light sources of four colors consisting of the violet LED 22, the blue LED 21, the green LED 23, and the red LED 24 are provided, such first semiconductor light sources of three colors are classified into two types such as a first group including the violet LED 22, the blue LED 21, and the green LED 23 (refer to Fig. 12A) and a second group including the blue LED 21, the green LED 23, and the red LED 24 (refer to Fig. 12B). Illumination light that is the second white light LW2 (refer to Fig. 1) can be generated by light emitted from each of the first semiconductor light sources of three colors of the first group. Illumination light that is the third white light LW3 (refer to Fig. 1) is generated by light emitted from each of the first semiconductor light sources of three colors of the second group.

In a case where the first semiconductor light sources of four colors described above are disposed in the light source device 10, and light of the three colors of the first group is used, this can be realized by controlling the light source control unit 81 to turn off the red LED 24. Similarly, in a case where the light of three colors of the second group is used, this can be realized by controlling the light source control unit 81 to turn off the violet LED 22.

In a case where the white light LW1, the second white light LW2, or the third white light LW3 is used as illumination light, it is possible to obtain an observation image having a natural tint that is easy for a doctor or the like to see. Therefore, the normal observation mode can be suitably used for normal observation such as screening of an observation target or endoscopy.

Specific examples of the second semiconductor light source are as follows. Since the second semiconductor light source can also be used as the third semiconductor light source, the following specific examples can also be applied to the third semiconductor light source. A device in which the second semiconductor light source is attached to the light source attachment unit 13 of the light source device 10 is the light source device 50 (refer to Fig. 4), and a device in which the second semiconductor light source of the light source device 50 is changed to the third semiconductor light source is the light source device 60. The following specific examples are applied to semiconductor light sources used in these devices.

The second semiconductor light source may have, as a wavelength range of emitted light, a wavelength range between adj acent wavelength ranges of pieces of light emitted from two first semiconductor light sources among the plurality of first semiconductor light sources. The fact of having a wavelength range between wavelength ranges of pieces of light emitted from two first semiconductor light sources specifically means a case where a wavelength range of light emitted from the second semiconductor light source includes a wavelength range between peak wavelengths or central wavelengths of the pieces of light emitted from the two first semiconductor light sources or a case where a peak wavelength or a central wavelength of light emitted from the second semiconductor light source is present in a wavelength range between peak wavelengths or central wavelengths of the pieces of light emitted from the two first semiconductor light sources. In this case, the wavelength ranges of the pieces of light emitted from the two first semiconductor light sources and the wavelength range of the light emitted from the second semiconductor light source may partially overlap.

In a case where the first semiconductor light sources are light sources four colors consisting of the blue LED 21, the violet LED 22, the green LED 23, and the red LED 24, and wavelength ranges of pieces of light emitted from the two first semiconductor light sources are adjacent to each other, there are three types such as the blue LED 21 and the green LED 23, the green LED 23 and the red LED 24, and the violet LED 22 and the blue LED 21. Therefore, in each of these three types, a wavelength range of light emitted from the second semiconductor light source may be set to a wavelength range between wavelength ranges of pieces of light emitted from two first semiconductor light sources of the three types.

The wavelength range of the light emitted from the second semiconductor light source may include a wavelength range between wavelength ranges of pieces of light emitted from the blue LED 21 and the green LED 23. Such a second semiconductor light source is, for example, a light blue semiconductor light source 25A having the light blue LED 25 as a light emitting element. The light blue LED 25 emits the light blue light LSB having a central wavelength of 470 nm and a wavelength range of 450 to 490 nm (refer to (A) of Fig. 5).

In a case where the second semiconductor light source is attached to the light source attachment unit 13, in the illumination light generation unit 12, the fourth dichroic filter 40 as the second multiplexing member is disposed in the second multiplexing member disposition portion 14. In this case, the characteristic data 40a of the fourth dichroic filter 40 in this case is a characteristic in which light in a wavelength range equal to or more than the green light LG is transmitted as shown in the transmission curve T, and light in a wavelength range equal to or less than the light blue light LSB is reflected as shown in the reflection curve R (refer to (B) of Fig. 5). That is, it is assumed that a cutoff wavelength of the fourth dichroic filter 40 is on a longer wavelength side than the wavelength range of the light blue light LSB and on a shorter wavelength side than the wavelength range of the green light LG.

It is preferable that the characteristic data 33a of the third dichroic filter 33 in this case is a characteristic of transmitting the light blue light LSB (refer to (C) of Fig. 5). In this case, the characteristic data 33a of the third dichroic filter 33 in this case is a characteristic in which light in a wavelength range equal to or more than the light blue light LSB is transmitted as shown in the transmission curve T, and light in which a wavelength range equal to or less than the blue light LB is reflected as shown in a reflection curve R. That is, it is assumed that a cutoff wavelength of the third dichroic filter 33 is on a longer wavelength side than the wavelength range of the blue light LB and on a shorter wavelength side than the wavelength range of the light blue light LSB.

In the light source device 10 before adding the second semiconductor light source, the characteristic data 33w of the third dichroic filter 33 may be a characteristic in which a cutoff wavelength is set in the vicinity of the central wavelength of 470 nm of the light blue light LSB to correspond to the first semiconductor light sources of four colors (refer to (B) of Fig. 2). In a case where the light blue LED 25 is added, in the light source device 50, the third dichroic filter 33 having the characteristic data 33w is replaced with the third dichroic filter 33 having the characteristic data 33a (refer to (C) of Fig. 5) that is a characteristic of sufficiently transmitting the light blue light LSB.

Illumination light having a multicolor spectrum consisting of a total of five colors, that is, four colors related to the blue LED 21, the violet LED 22, the green LED 23, and the red LED 24 of the first semiconductor light sources, and one color related to the light blue LED 25 of the second semiconductor light source, can be increased in an amount of light on the short wavelength side due to the light blue light LSB and can be used as illumination light suitable for fluorescence observation or the like, and is thus suitable for image enhanced endoscopy or the like using the illumination light.

The wavelength range of light emitted from the second semiconductor light source may include a wavelength range between wavelength ranges of pieces of light emitted from the green LED 23 and the red LED 24. Such a second semiconductor light source is, for example, a yellow semiconductor light source having a yellow LED 27 as a light emitting element. The yellow LED 27 emits yellow light LYE having a central wavelength of 580 to 600 nm and a wavelength range of 560 to 620 nm.

As shown in Fig. 13, the light source device 50 is a device in which the yellow LED 27 is attached to the light source attachment unit 13 in the light source device 10. The light source device 50 generates illumination light LX having a specific multicolor spectrum generated by using light emitted from each of the blue LED 21, the violet LED 22, the green LED 23, and the red LED 24, which are the first semiconductor light sources, and the yellow LED 27 that is the second semiconductor light source, and supplies the Illumination light LX to the light guide 41.

As shown in (A) of Fig. 14, illumination light having a multicolor spectrum emitted from the light source device 50 in this case has a wavelength range of light emitted the yellow LED 27 in a wavelength range between wavelength ranges of pieces of light emitted from the green LED 23 and the red LED 24. The light having the multicolor spectrum shown in (A) of Fig. 14 is an example of the illumination light LX, and the Illumination light LX having various multicolor spectra can be generated by adjusting, for example, a spectral spectrum or an amount of light emitted from each of the first semiconductor light sources or the second semiconductor light source.

The fourth dichroic filter 40 and the third dichroic filter 33 appropriately transmit or reflect the yellow light LYE emitted from the second semiconductor light source and light emitted from each of the plurality of first semiconductor light sources such that the illumination light LX is appropriately formed by the yellow light LYE emitted from the second semiconductor light source and the other light emitted from each of the plurality of first semiconductor light sources.

As shown in (B) of Fig. 14, characteristic data 40c of the fourth dichroic filter 40 in this case is a characteristic in which light in a wavelength range of the yellow light LYE is reflected as shown in a reflection curve R, and light in a wavelength range equal to or less than the green light LG and equal to or more than the red light LR as shown in a transmission curve T. That is, it is assumed that a cutoff wavelength of the fourth dichroic filter 40 is on a shorter wavelength side and a longer wavelength side than the wavelength range of the yellow light LYE.

The third dichroic filter 33 transmits the green light LG, the red light LR, and the yellow light LYE, and reflects the blue light LB and the violet light LV. As shown in (C) of Fig. 14, characteristic data 33c of the third dichroic filter 33 in this case is a characteristic in which light in a wavelength range equal to or more than the green light LG is transmitted as shown in a transmission curve T, and light in a wavelength range equal to or less than the blue light LB is reflected as shown in a reflection curve R.

In this case, it is preferable that the third dichroic filter 33 has a characteristic of transmitting the yellow light LY, but in a case where a cutoff wavelength is provided near 470 nm to reflect the wavelength range or less of the blue light LB and transmit the wavelength range or more of the green light LG, so as to correspond to the first semiconductor light sources of four colors in the light source device 10 before the second semiconductor light source is attached, the yellow light LY is transmitted, and thus it is not necessary to replace the third dichroic filter 33 in the light source device 10.

Illumination light having a multicolor spectrum consisting of a total of five colors, that is, four colors related to the blue LED 21, the violet LED 22, the green LED 23, and the red LED 24 of the first semiconductor light sources, and one color related to the yellow LED 27 of the second semiconductor light source, has a high ratio of the yellow light LYE absorbed by hemoglobin, and can be suitably used for image enhanced endoscopy or the like for facilitating viewing of blood vessels.

The wavelength range of light emitted from the second semiconductor light source may include a wavelength range between wavelength ranges of pieces of light emitted from the violet LED 22 and the blue LED 21. Such a second semiconductor light source is, for example, a blue-violet semiconductor light source having a blue-violet LED 28 as a light emitting element. The blue-violet LED 28 emits blue-violet light LBV having a central wavelength of 420 to 430 nm and a wavelength range of 410 to 440 nm, for example.

As shown in Fig. 15, the light source device 50 is a device in which the blue-violet LED 28 is attached to the light source attachment unit 13 in the light source device 10 in which the light source attachment unit 13 is provided in the middle of the optical path of the blue light LB. The light source device 50 generates illumination light LX having a specific multicolor spectrum generated by using light emitted from each of the blue LED 21, the violet LED 22, the green LED 23, and the red LED 24, which are the first semiconductor light sources, and the blue-violet LED 28 that is the second semiconductor light source, and supplies the Illumination light LX to the light guide 41.

As shown in (A) of Fig. 16, illumination light having a multicolor spectrum emitted from the light source device 50 in this case has a wavelength range of light emitted from the blue-violet LED 28 in a wavelength range between wavelength ranges of pieces of light emitted from the violet LED 22 and the blue LED 21. The blue-violet semiconductor light source may emit blue-violet light, for example, by causing the blue LED 21 to act on a red phosphor.

As shown in (B) of Fig. 16, characteristic data 40d of the fourth dichroic filter 40 in this case is a characteristic in which light in a wavelength range of the blue-violet light LBV is reflected as shown in a reflection curve R, and light in a wavelength range equal to or less than the violet light LV and equal to or more than the blue light LB is transmitted as shown in a transmission curve T. That is, it is assumed that a cutoff wavelength of the fourth dichroic filter 40 is on a shorter wavelength side and a longer wavelength side than the wavelength range of the blue-violet light LBV.

The third dichroic filter 33 transmits the blue light LB, the blue-violet light LBV, and the violet light LV, and reflects the green light LG and the red light LR. As shown in (C) of Fig. 16, characteristic data 33d of the third dichroic filter 33 in this case a characteristic in which light in a wavelength range equal to or more than the green light LG is reflected as shown in a reflection curve R, and light in a wavelength range equal to or less than the blue light LB is transmitted as shown in a transmission curve T.

In this case, it is preferable that the third dichroic filter 33 has a characteristic of transmitting the blue-violet light LBV, but in a case where a cutoff wavelength is provided near 470 nm to reflect the wavelength range or less of the blue light LB and transmit the wavelength range or more of the green light LG, so as to correspond to the first semiconductor light sources of four colors in the light source device 10 before the second semiconductor light source is attached, this third dichroic filter 33 is preferably replaced with the third dichroic filter 33 having the characteristic data 33d and a cutoff wavelength near 470 nm to transmit the wavelength range or less of the blue light LB and reflect the wavelength range or more of the green light LG.

Illumination light having a multicolor spectrum consisting of a total of five colors, that is, four colors related to the blue LED 21, the violet LED 22, the green LED 23, and the red LED 24 of the first semiconductor light sources, and one color related to the blue-violet LED 28 of the second semiconductor light source, can be suitably used for image enhanced endoscopy or the like in which the influence of blood is suppressed and the mucous membrane is easily seen.

The wavelength range of the light emitted from the second semiconductor light source may include a wavelength range in a wavelength range longer than the wavelength ranges of pieces of light emitted from the plurality of first semiconductor light sources. Specifically, the light emitted from the second semiconductor light source may include a wavelength range in an infrared range which is a longer wavelength range than the red light LR, or may include a wavelength range in a near-infrared range. Such a second semiconductor light source is, for example, the near-infrared light semiconductor light source 26A having the near-infrared light LED 26 as a light emitting element. The near-infrared light LED 26 emits near-infrared light LIR having a central wavelength of 780 nm and a wavelength range of 750 to 810 nm (refer to (A) of Fig. 9).

The multicolor spectrum in this case (refer to (A) of Fig. 9), the fourth dichroic filter 40 (refer to (B) of Fig. 9), and the third dichroic filter 33 (refer to (C) of Fig. 9) are described above.

Illumination light having a multicolor spectrum consisting of a total of five colors, that is, four colors related to the blue LED 21, the violet LED 22, the green LED 23, and the red LED 24 of the first semiconductor light sources, and one color related to the near-infrared light LIR of the second semiconductor light source, can be suitably used for image enhanced endoscopy in which blood vessels are identified with indocyanine green or the like.

The wavelength range of the light emitted from the second semiconductor light source may include a wavelength range in a wavelength range shorter than the wavelength ranges of pieces of light emitted from the plurality of first semiconductor light sources. Specifically, the light emitted from the second semiconductor light source may include a wavelength range in an ultraviolet range which is a shorter wavelength range than the violet light LV. Such a second semiconductor light source is, for example, an ultraviolet light semiconductor light source 29A having an ultraviolet light LED 29 as a light emitting element. The ultraviolet light LED 29 emits an ultraviolet LUV having a central wavelength of 350 nm and a wavelength range of 320 to 380 nm.

As shown in Fig. 17, the light source device 50 is a device in which the ultraviolet light LED 29 is attached to the light source attachment unit 13 in the light source device 10 in which the light source attachment unit 13 is provided in the middle of the optical path of the blue light LB. The light source device 50 generates illumination light LX having a specific multicolor spectrum generated by using light emitted from each of the blue LED 21, the violet LED 22, the green LED 23, and the red LED 24, which are the first semiconductor light sources, and the ultraviolet light LED 29 that is the second semiconductor light source, and supplies the Illumination light LX to the light guide 41. In the light source device 50 in this case, the fourth dichroic filter 40 disposed in the second multiplexing member disposition portion 14 reflects light in the wavelength range of the ultraviolet light LUV.

As shown in (A) of Fig. 18, illumination light of the multicolor spectrum emitted from the light source device 50 in this case has a wavelength range of light emitted from the ultraviolet light LED 29 in a wavelength range of a wavelength range shorter than the wavelength range of the violet light LV. The light having the multicolor spectrum shown in (A) of Fig. 18 is an example of the illumination light LX, and the Illumination light LX having various multicolor spectra can be generated by adjusting, for example, a spectral spectrum or an amount of light emitted from each of the first semiconductor light sources or the second semiconductor light source.

The fourth dichroic filter 40 and the third dichroic filter 33 appropriately transmit or reflect the ultraviolet light LUV emitted from the second semiconductor light source and light emitted from each of the plurality of first semiconductor light sources such that the illumination light LX is appropriately formed by the ultraviolet light LUV emitted from the second semiconductor light source and the other light emitted from each of the plurality of first semiconductor light sources.

As shown in (B) of Fig. 18, characteristic data 40e of the fourth dichroic filter 40 in this case is a characteristic in which light in a wavelength range equal to or more than the blue light LB is transmitted as shown in a transmission curve T, and light in a wavelength range equal to or less than the ultraviolet light LUV is reflected as shown in a reflection curve R. That is, it is assumed that a cutoff wavelength of the fourth dichroic filter 40 is in the vicinity of the wavelength range of the violet light LV.

The third dichroic filter 33 transmits the ultraviolet light LUV, the green light LG, and the red light LR, and reflects the blue light LB and the violet light LV. As shown in (C) of Fig. 18, characteristic data 33e of the third dichroic filter 33 in this case is a characteristic in which light in a wavelength range equal to or more than the green light LG and light in a wavelength range equal to or less than the ultraviolet light LUV are transmitted as shown in a transmission curve T, and light in a wavelength range equal to or less than the blue light LB and light in a wavelength range equal to or more than the violet light LV are reflected as shown in a reflection curve R.

In this case, it is preferable that the third dichroic filter 33 has a characteristic of transmitting the ultraviolet light LUV, but in a case where a cutoff wavelength is provided near 470 nm to reflect the wavelength range or less of the blue light LB and transmit the wavelength range or more of the green light LG, so as to correspond to the first semiconductor light sources of four colors in the light source device 10 before the second semiconductor light source is attached, this third dichroic filter 33 is preferably replaced with the third dichroic filter 33 having the characteristic data 33e and a cutoff wavelength near 470 nm and near 370 nm to transmit light in a wavelength range equal to or more than the green light LG and light in a wavelength range equal to or less than the ultraviolet light LUV and to reflect light in a wavelength range equal to or less than the blue light LB and equal to or more than the violet light LV.

Illumination light having a multicolor spectrum consisting of a total of five colors, that is, four colors related to the blue LED 21, the violet LED 22, the green LED 23, and the red LED 24 of the first semiconductor light sources and one color related to the ultraviolet light LED 29 of the second semiconductor light source, can be suitably used for image enhanced endoscopy using fluorescence.

In the above embodiments, hardware structures of processing units such as the central control unit 95, the DSP 94, the image processing unit 96, the frame memory 97, and the display control circuit included in the processor device 72 are various processors as described below. The various processors include a central processing unit (CPU) that is a general-purpose processor that executes software (program) to function as various processing units, a programmable logic device (PLD) such as a field programmable gate array (FPGA) of which a circuit configuration is changed after being manufactured, a dedicated electric circuit that is a processor having a circuit configuration specially designed to execute various processes, and the like.

One processing unit may be configured with one of these various processors, or may be configured with a combination of two or more processors of the same type or different types (for example, a combination of a plurality of FPGAs or a combination of a CPU and an FPGA). A plurality of processing units may be configured by one processor. As an example of configuring a plurality of processing units with one processor, first, there is a form in which one processor is configured by a combination of one or more CPUs and software, as typified by a computer used for a client or a server, and this processor functions as a plurality of processing units. Second, as typified by system on chip (SoC), there is a form in which a processor that realizes functions of the entire system including a plurality of processing units with one integrated circuit (IC) chip is used. As described above, the various processing units are configured by using one or more of the above various processors as a hardware structure.

The hardware structure of these various processors is, more specifically, an electric circuit (circuitry) in which circuit elements such as semiconductor elements are combined.

### Explanation of References

10, 50, 60: light source device
11: light source unit
12: illumination light generation unit
13: light source attachment unit
14: second multiplexing member disposition portion
21: blue LED
21A: blue semiconductor light source
22: violet LED
22A: violet semiconductor light source
23: green LED
23A: green semiconductor light source
24: red LED
24A: red semiconductor light source
25: light blue LED
25A: light blue semiconductor light source
26: near-infrared light LED
27: yellow LED
28: blue-violet LED
29: ultraviolet light LED
31: first dichroic filter
32: second dichroic filter
33: third dichroic filter
33a~33e, 33w: characteristic data
34: first collimator lens
35: second collimator lens
36: third collimator lens
37: fourth collimator lens
38: fifth collimator lens
39: condenser lens
40: fourth dichroic filter
40a~40e: characteristic data
41: light guide
41a: incident end
70: endoscope system
71: endoscope
71a: insertion part
71b: operating part
71c: bendable part
71d: tip part
71e: angle knob
71f: zoom operating part
72: processor device
73: display
74: operation input unit
75: universal cord
81: light source control unit
82: receptacle connector
83: light source connector
84: irradiation lens
84a: illumination window
85: objective optical system
85a: observation window
86: image pick-up element
86a: image pick-up surface
91: AFE
92: image pick-up control unit
93: communication connector
94: DSP
95: central control unit
96: image processing unit
97: frame memory
98: display control circuit
LB: blue light
LV: violet light
LG: green light
LR: red light
LW: white light
LSB: light blue light
LYE: yellow light
LBV: blue-violet light
LIR: near-infrared light
LUV: ultraviolet light
LX, LY: illumination light
T: transmission curve
R: reflection curve
ST110~ST200: step

## Claims

1. An endoscope light source device (10, 50, 60) that supplies illumination light to a light guide (41) of an endoscope, comprising:
a plurality of first semiconductor light sources (21A-24A) that emit pieces of light in wavelength ranges different from each other;
an illumination light generation unit (12) that generates the illumination light that is white light by using the pieces of light emitted from the plurality of first semiconductor light sources (21A-24A); and
a light source attachment unit (13) that is configured such that a second semiconductor light source (25) is attachable to and detachable from the light source attachment unit (13),
wherein the illumination light generation unit (12) generates the illumination light by using the pieces of light emitted from the plurality of first semiconductor light sources (21A-24A) and light emitted from the second semiconductor light source (25) in a case where the second semiconductor light source (25) is attached to the light source attachment unit (13);
wherein the illumination light generation unit (12) includes
a first multiplexing member (31-33) that multiplexes the pieces of light emitted from the plurality of first semiconductor light sources (21A-24A), and
a second multiplexing member disposition portion (14) in which a second multiplexing member (40) that reflects the light emitted from the second semiconductor light source (25) to multiplex light emitted from at least one of the plurality of first semiconductor light sources (21A-24A) with the light emitted from the second semiconductor light source (25) is disposed in a case where the second semiconductor light source (25) is attached; and
wherein the second multiplexing member disposition portion (14) is configured such that the second multiplexing member (40) is attachable to and detachable from the second multiplexing member disposition portion (14).

2. The endoscope light source device (10, 50, 60) according to claim 1,
wherein the light source attachment unit (13) is provided at a position where an optical path length of the light emitted from the second semiconductor light source (25) to the light guide (41) is shorter than an optical path length of the light emitted from each of the plurality of first semiconductor light sources (21A-24A) to the light guide (41).

3. The endoscope light source device (10, 50, 60) according to any one of claims 1 to 2,
wherein the plurality of first semiconductor light sources (21A-24A) include the first semiconductor light source (21A) that emits blue light or the first semiconductor light source (23A) that emits green light, and
the illumination light generation unit (12) is provided with the light source attachment unit (13) at a position where the light emitted from the second semiconductor light source (25) is multiplexed with the light emitted from the first semiconductor light source (21A) on an optical path of the blue light emitted from the first semiconductor light source or on an optical path of the green light emitted from the first semiconductor light source (23A).

4. The endoscope light source device (10, 50, 60) according to claim 1,
wherein the illumination light generation unit (12) generates the illumination light that is white light by using pieces of light emitted from the plurality of first semiconductor light sources (21A-24A) in a case where the second semiconductor light source (25) attached to the light source attachment unit (13) is detached from the light source attachment unit (13).

5. The endoscope light source device (10, 50, 60) according to claim 1,
wherein at least one first multiplexing member (31-33) is replaceable.

6. The endoscope light source device (10, 50, 60) according to any one of claims 1 to 5,
wherein each of the first semiconductor light sources (21A-24A) emits any one piece of light selected from among violet light, blue light, green light, and red light,
the plurality of first semiconductor light sources (21A-24A) include at least three first semiconductor light sources, and
a continuous wavelength range is formed by light emitted from each of the plurality of first semiconductor light sources (21A-24A).

7. The endoscope light source device (10, 50, 60) according to any one of claims 1 to 6,
wherein the plurality of first semiconductor light sources (21A-24A) include at least four first semiconductor light sources (21A-24A) that each emit any one piece of light selected from among violet light, blue light, green light, and red light, and
a continuous wavelength range is formed by light emitted from each of the plurality of first semiconductor light sources (21A-24A).

8. The endoscope light source device (10, 50, 60) according to any one of claims 1 to 7,
wherein, in a case where light emitted from each of two first semiconductor light sources (21A-24A) among the plurality of first semiconductor light sources (21A-24A) forms a continuous wavelength range, a wavelength range of the light emitted from the second semiconductor light source (25) includes a wavelength range between the wavelength ranges of the pieces of light emitted from the two first semiconductor light sources (21A-24A).

9. The endoscope light source device (10, 50, 60) according to any one of claims 1 to 7,
wherein the plurality of first semiconductor light sources (21A-24A) include the first semiconductor light source (21A) that emits blue light and the first semiconductor light source (23A) that emits green light, and
a wavelength range of the light emitted from the second semiconductor light source (25) includes a wavelength range between a wavelength range of the blue light emitted from the first semiconductor light source (21A) and a wavelength range of the green light emitted from the first semiconductor light source (23A).

10. The endoscope light source device (10, 50, 60) according to any one of claims 1 to 7,
wherein the plurality of first semiconductor light sources (21A-24A) include the first semiconductor light source (23A) that emits green light and the first semiconductor light source (24A) that emits red light, and
a wavelength range of the light emitted from the second semiconductor light source (25) includes a wavelength range between a wavelength range of the green light emitted from the first semiconductor light source (23A) and a wavelength range of the red light emitted from the first semiconductor light source (24A).

11. The endoscope light source device (10, 50, 60) according to any one of claims 1 to 7,
wherein the plurality of first semiconductor light sources (21A-24A) include the first semiconductor light source (22A) that emits violet light and the first semiconductor light source (21A) that emits blue light, and
a wavelength range of the light emitted from the second semiconductor light source (25) includes a wavelength range between a wavelength range of the violet light emitted from the first semiconductor light source (22A) and a wavelength range of the blue light emitted from the first semiconductor light source (21A).

12. The endoscope light source device (10, 50, 60) according to any one of claims 1 to 7,
wherein a wavelength range of the light emitted from the second semiconductor light source (25) includes a wavelength range in a wavelength range longer than the wavelength ranges of the pieces of light emitted from the plurality of first semiconductor light sources (21A-24A).

13. The endoscope light source device (10, 50, 60) according to any one of claims 1 to 7,
wherein a wavelength range of the light emitted from the second semiconductor light source (25) includes a wavelength range in a near-infrared range.

14. The endoscope light source device (10, 50, 60) according to any one of claims 1 to 7,
wherein a wavelength range of the light emitted from the second semiconductor light source (25) includes a wavelength range in a wavelength range shorter than the wavelength ranges of the pieces of light emitted from the plurality of first semiconductor light sources (21A-24A).

15. The endoscope light source device (10, 50, 60) according to any one of claims 1 to 7,
wherein a wavelength range of the light emitted from the second semiconductor light source (25) includes a wavelength range in an ultraviolet range.

16. An endoscope system comprising:
an endoscope that has a light guide (41) guiding illumination light; and
an endoscope light source device (10, 50, 60) that supplies the illumination light to the light guide (41), the endoscope light source device being according to any one of claims 1 to 15.

17. The endoscope system according to claim 16,
wherein the illumination light generation unit (12) generates the illumination light that is white light by using pieces of light emitted from the plurality of first semiconductor light sources (21A-24A) in a case where the second semiconductor light source (25) attached to the light source attachment unit (13) is detached from the light source attachment unit (13).

18. A method of changing illumination light in an endoscope light source device according to any one of claims 1 to 15 that supplies the illumination light to a light guide (41) of an endoscope, the method comprising:
a step of attaching the second semiconductor light source (25) to the light source attachment unit (13); and
a step of causing the illumination light generation unit (12) to generate the illumination light different from the white light by using the pieces of light emitted from the plurality of first semiconductor light sources (21A-24A) and light emitted from the second semiconductor light source (25).

19. The method of changing illumination light according to claim 18, the method further comprising:
a step of detaching the second semiconductor light source (25) from the light source attachment unit to which the second semiconductor light source (25) is attached;
a step of attaching a third semiconductor light source that emits light in a wavelength range different from that of the second semiconductor light source (25) to the light source attachment unit (13); and
a step of causing the illumination light generation unit (12) to generate the illumination light by using the pieces of light emitted from the plurality of the first semiconductor light sources (21A-24A) and the light emitted from the third semiconductor light source.

## Patentansprüche

1. Endoskop-Lichtquellenvorrichtung (10, 50, 60), die einem Lichtleiter (41) eines Endoskops Beleuchtungslicht zuführt, umfassend:
mehrere erste Halbleiterlichtquellen (21A-24A), die Lichtstücke in Wellenlängenbereichen emittieren, die sich voneinander unterscheiden;
eine Erzeugungseinheit (12) für Beleuchtungslicht, die das Beleuchtungslicht, das Weißlicht ist, durch Verwenden der Lichtstücke, die von den mehreren ersten Halbleiterlichtquellen (21A-24A) emittiert werden, erzeugt; und
eine Lichtquellen-Anbringungseinheit (13), die so konfiguriert ist, dass eine zweite Halbleiterlichtquelle (25) an der Lichtquellen-Anbringungseinheit (13) anbringbar und von dieser lösbar ist,
wobei die Erzeugungseinheit (12) für Beleuchtungslicht das Beleuchtungslicht durch Verwenden der Lichtstücke, die von den mehreren ersten Halbleiterlichtquellen (21A-15 24A) emittiert werden, und von Licht, das von der zweiten Halbleiterlichtquelle (25) emittiert wird, in einem Fall, in dem die zweite Halbleiterlichtquelle (25) an der Lichtquellen-Anbringungseinheit (13) angebracht ist, erzeugt;
wobei die Erzeugungseinheit (12) für Beleuchtungslicht enthält:
ein erstes Multiplexelement (31-33), das die von den mehreren ersten Halbleiterlichtquellen (21A-24A) emittierten Lichtstücke multiplext, und
einen zweiten Multiplexelement-Anordnungsabschnitt (14), in dem ein zweites Multiplexelement (40), das das von der zweiten Halbleiterlichtquelle (25) emittierte Licht reflektiert, um von mindestens einer von den mehreren ersten Halbleiterlichtquellen (21A-24A) emittiertes Licht mit dem von der zweiten Halbleiterlichtquelle (25) emittierten Licht zu multiplexen, in einem Fall, in dem die zweite Halbleiterlichtquelle (25) angebracht ist, angeordnet ist; und
wobei der zweite Multiplexelement-Anordnungsabschnitt (14) so konfiguriert ist, dass das zweite Multiplexelement (40) an dem zweiten Multiplexelement-Anordnungsabschnitt (14) anbringbar und von diesem lösbar ist.

2. Endoskop-Lichtquellenvorrichtung (10, 50, 60) nach Anspruch 1,
wobei die Lichtquellen-Anbringungseinheit (13) an einer Position vorgesehen ist, an der eine optische Weglänge des von der zweiten Halbleiterlichtquelle (25) emittierten Lichts zu dem Lichtleiter (41) kürzer als eine optische Weglänge des von jeder der mehreren ersten Halbleiterlichtquellen (21A-24A) emittierten Lichts zu dem Lichtleiter (41) ist.

3. Endoskop-Lichtquellenvorrichtung (10, 50, 60) nach einem der Ansprüche 1 bis 2,
wobei die mehreren ersten Halbleiterlichtquellen (21A-24A) die erste Halbleiterlichtquelle (21A), die Blaulicht emittiert, oder die erste Halbleiterlichtquelle (23A), die Grünlicht emittiert, enthalten, und
die Erzeugungseinheit (12) für Beleuchtungslicht mit der Lichtquellen-Anbringungseinheit (13) an einer Position versehen ist, an der das von der zweiten Halbleiterlichtquelle (25) emittierte Licht mit dem von der ersten Halbleiterlichtquelle (21A) emittierten Licht auf einem optischen Weg des von der ersten Halbleiterlichtquelle emittierten Blaulichts oder auf einem optischen Weg des von der ersten Halbleiterlichtquelle (23A) emittierten Grünlichts multiplext wird.

4. Endoskop-Lichtquellenvorrichtung (10, 50, 60) nach Anspruch 1,
wobei die Erzeugungseinheit (12) für Beleuchtungslicht das Beleuchtungslicht, das 5 Weißlicht ist, durch Verwenden von Lichtstücken, die von den mehreren ersten Halbleiterlichtquellen (21A-24A) emittiert werden, in einem Fall, in dem die zweite Halbleiterlichtquelle (25), die an der Lichtquellen-Anbringungseinheit (13) angebracht ist, von der Lichtquellen-Anbringungseinheit (13) gelöst wird, erzeugt.

5. Endoskop-Lichtquellenvorrichtung (10, 50, 60) nach Anspruch 1,
wobei mindestens ein erstes Multiplexelement (31-33) austauschbar ist.

6. Endoskop-Lichtquellenvorrichtung (10, 50, 60) nach einem der Ansprüche 1 bis 5,
wobei jede der ersten Halbleiterlichtquellen (21A-24A) ein beliebiges Lichtstück emittiert, das aus Violettlicht, Blaulicht, Grünlicht und Rotlicht ausgewählt wird,
die mehreren ersten Halbleiterlichtquellen (21A-24A) mindestens drei erste Halbleiterlichtquellen enthalten, und
ein durchgehender Wellenlängenbereich durch Licht, das von jeder der mehreren ersten Halbleiterlichtquellen (21A-24A) emittiert wird, gebildet wird.

7. Endoskop-Lichtquellenvorrichtung (10, 50, 60) nach einem der Ansprüche 1 bis 6,
wobei die mehreren ersten Halbleiterlichtquellen (21A-24A) mindestens vier erste Halbleiterlichtquellen (21A-24A) enthalten, die jeweils ein beliebiges Lichtstück emittieren, das aus Violettlicht, Blaulicht, Grünlicht und Rotlicht ausgewählt wird, und
ein durchgehender Wellenlängenbereich durch Licht, das von jeder der mehreren ersten Halbleiterlichtquellen (21A-24A) emittiert wird, gebildet wird.

8. Endoskop-Lichtquellenvorrichtung (10, 50, 60) nach einem der Ansprüche 1 bis 7,
wobei in einem Fall, in dem Licht, das von jeder von zwei ersten Halbleiterlichtquellen (21A-24A) unter den mehreren ersten Halbleiterlichtquellen (21A-24A) emittiert wird, einen durchgehenden Wellenlängenbereich bildet, ein Wellenlängenbereich des von der zweiten Halbleiterlichtquelle (25) emittierten Lichts einen Wellenlängenbereich zwischen den Wellenlängenbereichen der von den zwei ersten Halbleiterlichtquellen (21A-24A) emittierten Lichtstücke enthält.

9. Endoskop-Lichtquellenvorrichtung (10, 50, 60) nach einem der Ansprüche 1 bis 7,
wobei die mehreren ersten Halbleiterlichtquellen (21A-24A) die erste Halbleiterlichtquelle (21A), die Blaulicht emittiert, und die erste Halbleiterlichtquelle (23A), die Grünlicht emittiert, enthalten, und
ein Wellenlängenbereich des von der zweiten Halbleiterlichtquelle (25) emittierten Lichts einen Wellenlängenbereich zwischen einem Wellenlängenbereich des von der ersten Halbleiterlichtquelle (21A) emittierten Blaulichts und einem Wellenlängenbereich des von der ersten Halbleiterlichtquelle (23A) emittierten Grünlichts enthält.

10. Endoskop-Lichtquellenvorrichtung (10, 50, 60) nach einem der Ansprüche 1 bis 7,
wobei die mehreren ersten Halbleiterlichtquellen (21A-24A) die erste Halbleiterlichtquelle (23A), die Grünlicht emittiert, und die erste Halbleiterlichtquelle (24A), die Rotlicht emittiert, enthalten, und
ein Wellenlängenbereich des von der zweiten Halbleiterlichtquelle (25) emittierten Lichts einen Wellenlängenbereich zwischen einem Wellenlängenbereich des von der ersten Halbleiterlichtquelle (23A) emittierten Grünlichts und einem Wellenlängenbereich des von der ersten Halbleiterlichtquelle (24A) emittierten Rotlichts enthält.

11. Endoskop-Lichtquellenvorrichtung (10, 50, 60) nach einem der Ansprüche 1 bis 7,
wobei die mehreren ersten Halbleiterlichtquellen (21A-24A) die erste Halbleiterlichtquelle (22A), die Violettlicht emittiert, und die erste Halbleiterlichtquelle (21A), die Blaulicht emittiert, enthalten, und
ein Wellenlängenbereich des von der zweiten Halbleiterlichtquelle (25) emittierten Lichts einen Wellenlängenbereich zwischen einem Wellenlängenbereich des von der ersten Halbleiterlichtquelle (22A) emittierten Violettlichts und einem Wellenlängenbereich des von der ersten Halbleiterlichtquelle (21A) emittierten Blaulichts enthält.

12. Endoskop-Lichtquellenvorrichtung (10, 50, 60) nach einem der Ansprüche 1 bis 7,
wobei ein Wellenlängenbereich des von der zweiten Halbleiterlichtquelle (25) emittierten Lichts einen Wellenlängenbereich in einem Wellenlängenbereich, der länger als die Wellenlängenbereiche der von den mehreren ersten Halbleiterlichtquellen (21A-24A) emittierten Lichtstücke ist, enthält.

13. Endoskop-Lichtquellenvorrichtung (10, 50, 60) nach einem der Ansprüche 1 bis 7,
wobei ein Wellenlängenbereich des von der zweiten Halbleiterlichtquelle (25) emittierten Lichts einen Wellenlängenbereich in einem Nahinfrarotbereich enthält.

14. Endoskop-Lichtquellenvorrichtung (10, 50, 60) nach einem der Ansprüche 1 bis 7,
wobei ein Wellenlängenbereich des von der zweiten Halbleiterlichtquelle (25) emittierten Lichts einen Wellenlängenbereich in einem Wellenlängenbereich, der kürzer als die Wellenlängenbereiche der von den mehreren ersten Halbleiterlichtquellen (21A-24A) emittierten Lichtstücke ist, enthält.

15. Endoskop-Lichtquellenvorrichtung (10, 50, 60) nach einem der Ansprüche 1 bis 7,
wobei ein Wellenlängenbereich des von der zweiten Halbleiterlichtquelle (25) emittierten Lichts einen Wellenlängenbereich in einem Ultraviolettbereich enthält.

16. Endoskopsystem, umfassend:
ein Endoskop, das einen Lichtleiter (41), der Beleuchtungslicht leitet, aufweist; und
eine Endoskop-Lichtquellenvorrichtung (10, 50, 60), die dem Lichtleiter (41) das Beleuchtungslicht zuführt, wobei die Endoskop-Lichtquellenvorrichtung nach einem der Ansprüche 1 bis 15 ist.

17. Endoskopsystem nach Anspruch 16,
wobei die Erzeugungseinheit (12) für Beleuchtungslicht das Beleuchtungslicht, das Weißlicht ist, durch Verwenden von Lichtstücken, die von den mehreren ersten Halbleiterlichtquellen (21A-24A) emittiert werden, in einem Fall, in dem die zweite Halbleiterlichtquelle (25), die an der Lichtquellen-Anbringungseinheit (13) angebracht ist, von der Lichtquellen-Anbringungseinheit (13) gelöst wird, erzeugt.

18. Verfahren des Änderns von Beleuchtungslicht bei einer Endoskop-Lichtquellenvorrichtung nach einem der Ansprüche 1 bis 15, die dem Lichtleiter (41) eines Endoskops das Beleuchtungslicht zuführt, wobei das Verfahren umfasst:
einen Schritt des Anbringens der zweiten Halbleiterlichtquelle (25) an der Lichtquellen-Anbringungseinheit (13); und
einen Schritt des Veranlassens, dass die Erzeugungseinheit (12) für Beleuchtungslicht das Beleuchtungslicht, das sich von dem Weißlicht unterscheidet, durch Verwenden der Lichtstücke, die von den mehreren ersten Halbleiterlichtquellen (21A-24A) emittiert werden, und von Licht, das von der zweiten Halbleiterlichtquelle (25) emittiert wird, erzeugt.

19. Verfahren des Änderns von Beleuchtungslicht nach Anspruch 18, wobei das Verfahren ferner umfasst:
einen Schritt des Lösens der zweiten Halbleiterlichtquelle (25) von der Lichtquellen-Anbringungseinheit, an der die zweite Halbleiterlichtquelle (25) angebracht ist;
einen Schritt des Anbringens einer dritten Halbleiterlichtquelle, die Licht in einem Wellenlängenbereich emittiert, der sich von dem der zweiten Halbleiterlichtquelle (25) unterscheidet, an der Lichtquellen-Anbringungseinheit (13); und
einen Schritt des Veranlassens, dass die Erzeugungseinheit (12) für Beleuchtungslicht das Beleuchtungslicht durch Verwenden der Lichtstücke, die von den mehreren ersten Halbleiterlichtquellen (21A-24A) emittiert werden, und von dem Licht, das von der dritten Halbleiterlichtquelle emittiert wird, erzeugt.

## Revendications

1. Dispositif de source de lumière d'endoscope (10, 50, 60) qui fournit une lumière d'éclairage à un guide de lumière (41) d'un endoscope, comprenant :
une pluralité de premières sources de lumière semi-conductrices (21A-24A) qui émettent des faisceaux lumineux dans des plages de longueurs d'onde différentes les unes des autres ;
une unité de génération de lumière d'éclairage (12) qui génère la lumière d'éclairage qui est une lumière blanche en utilisant les faisceaux lumineux émis par la pluralité de premières sources de lumière semi-conductrices (21A-24A) ; et
une unité de fixation de source de lumière (13) qui est configurée de sorte qu'une deuxième source de lumière semi-conductrice (25) puisse être fixée à et détachée de l'unité de fixation de source de lumière (13),
dans lequel l'unité de génération de lumière d'éclairage (12) génère la lumière d'éclairage en utilisant les faisceaux lumineux émis par la pluralité de premières sources de lumière semi-conductrices (21A-24A) et la lumière émise par la deuxième source de lumière semi-conductrice (25) dans un cas où la deuxième source de lumière semi-conductrice (25) est fixée à l'unité de fixation de source de lumière (13) ;
dans lequel l'unité de génération de lumière d'éclairage (12) inclut :
un premier élément de multiplexage (31-33) qui multiplexe les faisceaux lumineux émis par la pluralité de premières sources de lumière semi-conductrices (21A-24A), et
une partie de disposition de deuxième élément de multiplexage (14) dans laquelle est disposé un deuxième élément de multiplexage (40) qui réfléchit la lumière émise par la deuxième source de lumière semi-conductrice (25) pour multiplexer la lumière émise par au moins une de la pluralité de premières sources de lumière semi-conductrices (21A-24A) avec la lumière émise par la deuxième source de lumière semi-conductrice (25), dans un cas où la deuxième source de lumière semi-conductrice (25) est fixée ; et
dans lequel la partie de disposition de deuxième élément de multiplexage (14) est configurée de sorte que le deuxième élément de multiplexage (40) puisse être fixé à et détaché de la partie de disposition de deuxième élément de multiplexage (14).

2. Dispositif de source de lumière d'endoscope (10, 50, 60) selon la revendication 1,
dans lequel l'unité de fixation de source de lumière (13) est fournie sur une position où une longueur de trajet optique de la lumière émise par la deuxième source de lumière semi-conductrice (25) au guide de lumière (41) est plus courte qu'une longueur de trajet optique de la lumière émise par chacune de la pluralité de premières sources de lumière semi-conductrices (21A-24A) au guide de lumière (41).

3. Dispositif de source de lumière d'endoscope (10, 50, 60) selon l'une quelconque des revendications 1 à 2,
dans lequel la pluralité de premières sources de lumière semi-conductrices (21A-24A) inclut la première source de lumière semi-conductrice (21A) qui émet une lumière bleue ou la première source de lumière semi-conductrice (23A) qui émet une lumière verte, et
l'unité de génération de lumière d'éclairage (12) est pourvue de l'unité de fixation de source de lumière (13) sur une position où la lumière émise par la deuxième source de lumière semi-conductrice (25) est multiplexée avec la lumière émise par la première source de lumière semi-conductrice (21A) sur un trajet optique de la lumière bleue émise par la première source de lumière semi-conductrice ou sur un trajet optique de la lumière verte émise par la première source de lumière semi-conductrice (23A).

4. Dispositif de source de lumière d'endoscope (10, 50, 60) selon la revendication 1,
dans lequel l'unité de génération de lumière d'éclairage (12) génère la lumière d'éclairage qui est une lumière blanche en utilisant des faisceaux lumineux émis par la pluralité de premières sources de lumière semi-conductrices (21A-24A) dans un cas où la deuxième source de lumière semi-conductrice (25) fixée à l'unité de fixation de source de lumière (13) est détachée de l'unité de fixation de source de lumière (13).

5. Dispositif de source de lumière d'endoscope (10, 50, 60) selon la revendication 1,
dans lequel au moins un premier élément de multiplexage (31-33) est remplaçable.

6. Dispositif de source de lumière d'endoscope (10, 50, 60) selon l'une quelconque des revendications 1 à 5,
dans lequel chacune des premières sources de lumière semi-conductrices (21A-24A) émet un quelconque faisceau lumineux choisi parmi une lumière violette, une lumière bleue, une lumière verte et une lumière rouge,
la pluralité de premières sources de lumière semi-conductrices (21A-24A) inclut au moins trois premières sources de lumière semi-conductrices, et
une plage de longueurs d'onde continue est formée par la lumière émise par chacune de la pluralité de premières sources de lumière semi-conductrices (21A-24A).

7. Dispositif de source de lumière d'endoscope (10, 50, 60) selon l'une quelconque des revendications 1 à 6,
dans lequel la pluralité de premières sources de lumière semi-conductrices (21A-24A) inclut au moins quatre premières sources de lumière semi-conductrices (21A-24A) qui émettent chacune un quelconque faisceau lumineux choisi parmi une lumière violette, une lumière bleue, une lumière verte, et une lumière rouge, et
une plage de longueurs d'onde continue est formée par la lumière émise par chacune de la pluralité de premières sources de lumière semi-conductrices (21A-24A).

8. Dispositif de source de lumière d'endoscope (10, 50, 60) selon l'une quelconque des revendications 1 à 7,
dans lequel, dans un cas où la lumière émise par chacune de deux premières sources de lumière semi-conductrices (21A-24A) parmi la pluralité de premières sources de lumière semi-conductrices (21A-24A) forme une plage de longueurs d'onde continue, une plage de longueurs d'onde de la lumière émise par la deuxième source de lumière semi-conductrice (25) inclut une plage de longueurs d'onde située entre les plages de longueurs d'onde des faisceaux lumineux émis par les deux premières sources de lumière semi-conductrices (21A-24A).

9. Dispositif de source de lumière d'endoscope (10, 50, 60) selon l'une quelconque des revendications 1 à 7,
dans lequel la pluralité de premières sources de lumière semi-conductrices (21A-24A) inclut la première source de lumière semi-conductrice (21A) qui émet une lumière bleue et la première source de lumière semi-conductrice (23A) qui émet une lumière verte, et
une plage de longueurs d'onde de la lumière émise par la deuxième source de lumière semi-conductrice (25) inclut une plage de longueurs d'onde située entre une plage de longueurs d'onde de la lumière bleue émise par la première source de lumière semi-conductrice (21A) et une plage de longueurs d'onde de la lumière verte émise par la première source de lumière semi-conductrice (23A).

10. Dispositif de source de lumière d'endoscope (10, 50, 60) selon l'une quelconque des revendications 1 à 7,
dans lequel la pluralité de premières sources de lumière semi-conductrices (21A-24A) inclut la première source de lumière semi-conductrice (23A) qui émet une lumière verte et la première source de lumière semi-conductrice (24A) qui émet une lumière rouge, et
une plage de longueurs d'onde de la lumière émise par la deuxième source de lumière semi-conductrice (25) inclut une plage de longueurs d'onde située entre une plage de longueurs d'onde de la lumière verte émise par la première source de lumière semi-conductrice (23A) et une plage de longueurs d'onde de la lumière rouge émise par la première source de lumière semi-conductrice (24A).

11. Dispositif de source de lumière d'endoscope (10, 50, 60) selon l'une quelconque des revendications 1 à 7,
dans lequel la pluralité de premières sources de lumière semi-conductrices (21A-24A) inclut la première source de lumière semi-conductrice (22A) qui émet une lumière violette et la première source de lumière semi-conductrice (21A) qui émet une lumière bleue, et
une plage de longueurs d'onde de la lumière émise par la deuxième source de lumière semi-conductrice (25) inclut une plage de longueurs d'onde située entre une plage de longueurs d'onde de la lumière violette émise par la première source de lumière semi-conductrice (22A) et une plage de longueurs d'onde de la lumière bleue émise par la première source de lumière semi-conductrice (21A).

12. Dispositif de source de lumière d'endoscope (10, 50, 60) selon l'une quelconque des revendications 1 à 7,
dans lequel une plage de longueurs d'onde de la lumière émise par la deuxième source de lumière semi-conductrice (25) inclut une plage de longueurs d'onde située dans une plage de longueurs d'onde plus longue que les plages de longueurs d'onde des faisceaux lumineux émis par la pluralité de premières sources de lumière semi-conductrices (21A-24A).

13. Dispositif de source de lumière d'endoscope (10, 50, 60) selon l'une quelconque des revendications 1 à 7,
dans lequel une plage de longueurs d'onde de la lumière émise par la deuxième source de lumière semi-conductrice (25) inclut une plage de longueurs d'onde dans une plage infrarouge proche.

14. Dispositif de source de lumière d'endoscope (10, 50, 60) selon l'une quelconque des revendications 1 à 7,
dans lequel une plage de longueurs d'onde de la lumière émise par la deuxième source de lumière semi-conductrice (25) inclut une plage de longueurs d'onde située dans une plage de longueurs d'onde plus courte que les plages de longueurs d'onde des faisceaux lumineux émis par la pluralité de premières sources de lumière semi-conductrices (21A-24A).

15. Dispositif de source de lumière d'endoscope (10, 50, 60) selon l'une quelconque des revendications 1 à 7,
dans lequel une plage de longueurs d'onde de la lumière émise par la deuxième source de lumière semi-conductrice (25) inclut une plage de longueurs d'onde dans une plage ultraviolette.

16. Système d'endoscope comprenant :
un endoscope qui comporte un guide de lumière (41) guidant une lumière d'éclairage ; et
un dispositif de source de lumière d'endoscope (10, 50, 60) qui fournit la lumière d'éclairage au guide de lumière (41), le dispositif de source de lumière d'endoscope étant selon l'une quelconque des revendications 1 à 15.

17. Système d'endoscope selon la revendication 16,
dans lequel l'unité de génération de lumière d'éclairage (12) génère la lumière d'éclairage qui est une lumière blanche en utilisant des faisceaux lumineux émis par la pluralité de premières sources de lumière semi-conductrices (21A-24A) dans un cas où la deuxième source de lumière semi-conductrice (25) fixée à l'unité de fixation de source de lumière (13) est détachée de l'unité de fixation de source de lumière (13).

18. Procédé de modification de lumière d'éclairage dans un dispositif de source de lumière d'endoscope selon l'une quelconque des revendications 1 à 15 qui fournit la lumière d'éclairage à un guide de lumière (41) d'un endoscope, le procédé comprenant :
une étape consistant à fixer la deuxième source de lumière semi-conductrice (25) à l'unité de fixation de source de lumière (13) ; et
une étape consistant à amener l'unité de génération de lumière d'éclairage (12) à générer la lumière d'éclairage différente de la lumière blanche en utilisant les faisceaux lumineux émis par la pluralité de premières sources de lumière semi-conductrices (21A-24A) et la lumière émise par la deuxième source de lumière semi-conductrice (25).

19. Procédé de modification de lumière d'éclairage selon la revendication 18, le procédé comprenant en outre :
une étape consistant à détacher la deuxième source de lumière semi-conductrice (25) de l'unité de fixation de source de lumière à laquelle la deuxième source de lumière semi-conductrice (25) est fixée ;
une étape consistant à fixer une troisième source de lumière semi-conductrice qui émet de la lumière dans une plage de longueurs d'onde différente de celle de la deuxième source de lumière semi-conductrice (25) à l'unité de fixation de source de lumière (13) ; et
une étape consistant à amener l'unité de génération de lumière d'éclairage (12) à générer la lumière d'éclairage en utilisant les faisceaux lumineux émis par la pluralité des premières sources de lumière semi-conductrices (21A-24A) et la lumière émise par la troisième source de lumière semi-conductrice.
